(19) Europäisches Patentamt
European Patent Office
Office européen des brevets

(11) EP 1 007 044 B1

(12) **EUROPEAN PATENT SPECIFICATION**

(45) Date of publication and mention of the grant of the patent:
**18.07.2007 Bulletin 2007/29**

(21) Application number: **98903275.0**

(22) Date of filing: **11.02.1998**

(51) Int Cl.:
*A61K 31/445* (2006.01) *A61K 31/505* (2006.01)

(86) International application number:
**PCT/IL1998/000070**

(87) International publication number:
**WO 1998/034613 (13.08.1998 Gazette 1998/32)**

(54) **QUINAZOLINONE-CONTAINING PHARMACEUTICAL COMPOSITIONS FOR PREVENTION OF NEOVASCULARIZATION AND FOR TREATING MALIGNANCIES**

QUINAZOLINENON-ENTHALTENDE PHARMAZEUTISCHE ZUSAMMENSETZUNGEN ZUR VERHÜTUNG VON GEFÄSSNEUBILDUNG UND ZUR BEHANDLUNG VON BÖSARTIGEN NEUBILDUNGEN

COMPOSITIONS PHARMACEUTIQUES CONTENANT DE LA QUINAZOLINONE ET DESTINEES A LA PREVENTION DE LA NEOFORMATION DE VAISSEAUX SANGUINS ET AU TRAITEMENT D'AFFECTIONS MALIGNES

(84) Designated Contracting States:
**AT BE CH DE DK ES FI FR GB GR IE IT LI LU MC NL PT SE**

(30) Priority: **11.02.1997 US 797703**

(43) Date of publication of application:
**14.06.2000 Bulletin 2000/24**

(73) Proprietors:
- **Agricultural Research Organization, Ministry of Agriculture**
  **Bet Dagan 50250 (IL)**
- **HADASIT MEDICAL RESEARCH SERVICES & DEVELOPMENT CO., LTD.**
  **Jerusalem 91120 (IL)**

(72) Inventors:
- **PINES, Mark**
  **76308 Rehovot (IL)**
- **NAGLER, Arnon**
  **74381 Jerusalem (IL)**
- **VLODAVSKY, Israel**
  **90805 Mevaseret Zion (IL)**
- **MIAO, Hua-Quan**
  **91120 Jerusalem (IL)**

(74) Representative: **Hartley, Andrew Philip et al**
**Mathisen, Macara & Co**
**The Coach House**
**6-8 Swakeleys Road**
**Ickenham, Uxbridge UB10 8BZ (GB)**

(56) References cited:
**WO-A-97/06805** **US-A- 5 449 678**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

FIELD AND BACKGROUND OF THE INVENTION

The present invention relates to the use of Halofuginone and pharmaceutically acceptable salts thereof for preparing a medicament for treating a tumor selected from the group consisting of breast cancer, lung cancer, bladder cancer, rhabdomyosarcoma, angiosarcoma, adenocarcinoma of the colon, prostate or pancreas, squamous cell carcinoma of the cervix, ovarian cancer, malignant fibrous histiocytoma, skin cancer astrocytoma, glioma and hepatocellularcarcinoma.

Malignancies are characterized by the growth and spread of tumors. A number of factors are important in the progression of this disease. These factors include the ability of the tumor cells to evade mechanisms for apoptosis (programmed cell death), angiogenesis, and the ability of malignant tumor cells to metastasize. Any one of these factors is a potential target for therapeutic intervention. Treatments which are able to affect two or more of these factors would be particularly desirable.

Angiogenesis is a complex process in which capillary blood vessels grow in an ordered sequence of events [J. Folkman and M. Klagsbrun, Science, Vol. 235, pp 442-447 (1987); J. Folkman and Y. Shing, J. Biol. Chem., Vol. 267, pp. 10931-10934 (1992)]. Angiogenesis is related to the development and progression of malignancies as follows. Once a tumor has started, every increase in tumor cell population must be preceded by an increase in new capillaries that converge on the tumor and supply the cells with oxygen and nutrients [J. Folkman, Perspect. in Biol. and Med., Vol 29, p. 10-36 (1985); J. Folkman, J. Natl. Cancer Inst., Vol. 82, pp. 4-6 (1989); N. Weidner, et al., Amer. J. Pathol., Vol. 143, pp. 401-409 (1993)]. Tumors may thus remain relatively harmless and confined to their tissue of origin, as long as an accompanying angiogenic program is prevented from being activated. Since the angiogenesis-dependent step in tumor progression is shared by solid tumors of all etiologies, the ability to inhibit tumor-associated angiogenesis is a most promising approach in combating cancer [M.S. O'Reilly, et al., Cell, Vol. 79, pp. 316-328 (1994)].

A substantial body of experimental evidence supports the hypothesis that tumor angiogenesis is fundamental for the growth and metastasis of solid tumors [J. Folkman, ibid. (1989); N. Weidner, et al., ibid. (1993); M.S. O'Reilly, et al., ibid. (1994); N. Weidner, et al., N. Eng. J. Med., Vol. 324, pp. 1-8 (1991)]. Indeed, the majority of solid tumors are not even clinically detectable until after the occurrence of neovascularization, whose induction in solid tumors is mediated by one or more angiogenic factors [J. Folkman, ibid. (1987); J. Folkman and Y. Shing, ibid. (1992)].

Furthermore, angiogenesis is also important in a number of other pathological processes, including arthritis, psoriasis, diabetic retinopathy, chronic inflammation, scleroderma, hemangioma, retrolental fibroplasia and abnormal capillary proliferation in hemophiliac joints, prolonged menstruation and bleeding, and other disorders of the female reproductive system [J. Folkman, Nature Medicine, Vol 1, p. 27-31, (1995); J.W. Miller, et al., J. Pathol., Vol. 145, pp. 574-584 (1994); A.P. Adamid, et al., Amer. J. Ophthal., Vol. 118, pp. 445-450 (1994); K. Takahashi, et al., J. Clin. Invest., Vol. 93, pp. 2357-2364 (1994); D.J. Peacock, et al., J. Exp. Med., Vol. 175, pp. 1135-1138 (1992); B.J. Nickoloff, et al., Amer. J. Pathol., Vol. 44, pp. 820-828 (1994); J. Folkman, Steroid Hormones and Uterine Bleeding, N.J. Alexander and C. d'Arcangues, Eds., American Association for the Advancement of Science Press, Washington, D.C., U.S.A., pp. 144-158 (1992)].

Thus, clearly new medicaments which would specifically block the mechanism of angiogenesis would be medically useful for the treatment of a number of different diseases. The basic mechanism of angiogenesis is as follows. Briefly, when a new capillary sprout grows from the side of the venule, endothelial cells degrade basement membrane, migrate toward an angiogenic source, proliferate, form a lumen, join the tips of two sprouts to generate a capillary loop, and manufacture new basement membrane [J. Folkman, Perspectives in Biology and Medicine, Vol. 29, pp. 1-36 (1985)].

Degradation and remodeling of the ECM are essential processes for the mechanism of angiogenesis. In addition, ECM components synthesized by endothelial cells such as collagens, laminin, thrombospondin, fibronectin and SPARC, function to regulate endothelial cell growth, migration and shape [J. Bischoff, Trends Cell Biol., No. 5, pp. 69-74 (1995)]. Bovine aortic endothelial cells (BAE) undergoing sprouting and tube formation synthesize type I collagen and SPARC. Type I collagen may direct migration and assembly of the BAE cells [M.L. Iruela-Arispe, et al., Lab. Invest., No. 64, pp. 174-186 (1991). Furthermore, exogenous type I collagen was found to promote rapid tube formation by confluent human dermal microvascular endothelial cells [C.J. Jackson and K.L. Jenkins, Exp. Cell Res., No. 192, pp. 319-323 (1991)]. The tubes thus formed contained collagen fibrils in the luminal spaces, suggesting that the endothelial cells use the fibrils to fold and align into tube structures.

The role of collagens in angiogenesis has been investigated by several groups. Metabolic inhibition of synthesis of collagen types I and IV inhibits capillary formation on the chorioallantoic membrane (CAM) assay (Maragoudakis, M.E. et al.., Int. J. Radiat. Biol, Vol. 60, 54-9, 1991). Furthermore, experimentally induced angiogenesis on the CAM was found to be associated with the deposition of large amounts of collagen. Endothelial cell migration from aortic explants within collagen gels is inhibited if grown in the presence of the proline analog cis-hydroxyproline, once again indicating the need for normal collagen synthesis (Nicosia, R.F. et al., In Vitro Cell Dev. Biol., Vol. 27A, 961-6, 1991).

*In vitro* studies suggest that co-expression of type I collagen and the calcium-binding protein SPARC (secreted protein, acidic, and rich in cysteine) is initiated when bovine aortic endothelial cells BAEC undergo sprouting angiogenesis (Iruela-Arispe, M.L. et al., Arterioscler. Thromb., Vol. 11, p. 805-15, 1991). Type VIII collagen is also synthesized during sprouting and has been localized to proliferating endothelial cells within endothelial cords. Histological examination of normal, tumor, and experimentally induced angiogenesis has extended the demonstration that type I and type VIII collagens are localized within endothelial cords and tubes (Kittelberger, R. et al., Connect. Tissue Res., Vol. 24, p. 303-18, 1990). It was reported that when BAEC are exposed to angiogenic hyaluronic acid (HA) oligosaccharides, type I collagen synthesis is up-regulated 4.5-fold and synthesis of type VIII collagen is enhanced 5.8-fold within 12 h. Type I collagen is believed to aid endothelial cell migration, while type VIII collagen is considered to be involved in endothelial cell migration and tube formation.

In addition to the production of collagen, in order to extend a capillary blood vessel, interactions must occur between ECM components and the surrounding matrix molecules, which provide a scaffold for the ECM components of the new vessel [Brooks, P.C. et al., Cell, Vol 79, p. 1157-1164, (1994)]. Disruption of these cell-matrix interactions induced apoptosis in human endothelial cells. Integrin $\alpha_2\beta_3$, which has an enhanced expression in angiogenic vascular cells, was also found to promote a survival signal, since inhibitors of this integrin caused unscheduled apoptosis and disintegration of newly formed blood vessels.

In order to treat angiogenesis-related diseases, several inhibitors of the above-described mechanism of angiogenesis are being studied, including platelet factor 4, the fumagillin derivative AGM 1470, Interferon $\alpha_2$a, thrombospondin, angiostatic steroids, and angiostatin [J. Folkman, ibid., (1995); M.S. O'Reilly, et al., ibid. (1994); V. Castle, et al., J. Clin. Invest., Vol. 87, pp. 1883-1888; D. Ingber, et al., Nature, Vol. 348, pp. 555-557]. All of these tested prior art compounds have disadvantages. For example, endostatin and angiostatin are proteins, so that they have all of the disadvantages of proteins, including the requirement for being administered parenterally. Therefore, a non-protein inhibitor which would selectively block the underlying mechanism of angiogenesis without adversely affecting other physiological functions, and which could be administered by many different routes, would be extremely useful. Such inhibitors would be useful for the treatment of a wide variety of diseases, such as malignancies, which include angiogenesis as part of the pathological process.

As noted above, degradation and remodeling of the ECM are essential processes for the mechanism of angiogenesis. Such processes involve the synthesis of a number of components of the ECM, such as collagen. A number of modulators of collagen metabolism have been examined for their affects on angiogenesis [Nakajima M. et al., Cancer Res., Vol. 9, p. 1698-1706, 1989; Turpeenniemi-Hujanen, T. et al., J. Natl. Cancer Inst., Vol. 75, p. 99-103, 1985; Monteagudo, C. et al., Amer. J. Pathol., Vol. 136, p. 585-592, 1990]. Regression of growing capillaries in the chick embryo was induced by proline analogs such as L-azetidine-2-carboxylic acid, *cis*-hydroxyproline, *d*L-3,4-dehydroxyproline and thioproline. These compounds and $\alpha,\alpha$-dipyridyl, an inhibitor of prolyl hydroxylase, all interfere with triple helix formation and prevent collagen deposition. $\beta$-amino-propionitrile, an inhibitor of collagen cross linking, was also anti-angiogenic, although $\beta$-methyl-*d*-xyloside, an inhibitor of glycosaminoglycan deposition, did not have antiangiogenic activity [Herron, G.S. et al., J. Biol. Chem., Vol. 261, p. 2814-2818, 1986; Reich, R. et al., Clin. and Exp. Metastasis, Vol. 13, p. 134-140, 1995]. Co-administration of suboptimal doses of collagen modulators with angiostatic steroids and/or heparin potentiated inhibition of angiogenesis.

In addition, tumors themselves, and in particular solid tumors, rely upon the synthesis and deposition of collagen for continued growth and progression, in addition to the requirement of collagen for angiogenesis to support tumor growth. Solid tumors are composed of two distinct but interdependent compartments: the malignant cells themselves and the stroma, which supports tumor growth by increased ECM and collagen type I synthesis (Folkman, J., Seminars in Cancer Biology, Vol. 3, p. 56-71, 1992). The stroma is essential for tumor growth since it supports the vascular supply required by tumors for obtaining nutrients, gas exchange and waste disposal. The great bulk of tumor stroma, up to 90% in some cases, is composed of interstitial connective tissue. This tissue includes structural proteins such as interstitial collagens, including collagen types I and III, fibrin, fibronectin, tenascin, elastin and sulfated proteoglycans (Dvorak, H.F., N. Eng. J. Med., Vol. 315, p. 1650-1659, 1986; Yeo, T.K., "Tumor Stroma" in Diagnostic Immunopathology, Ed. by Colvin, R.B. et al., Raven Press, New York, p. 985-696). General inhibitors of collagen formation were examined for their effect on tumor growth, and were found to inhibit tumor growth in mice but proved too toxic for long-term safe administration. Thus, currently available inhibitors of collagen synthesis and deposition are not suitable for the treatment of malignancies.

In addition, many other available inhibitors of collagen synthesis and deposition, although not examined for their effects on angiogenesis or tumor growth, are generally undesirable because they lack specificity for the collagen metabolic pathway. Thus, many currently available drugs have deleterious side effects.

For example, cytotoxic drugs have been used in an attempt to slow the proliferation of collagen-producing fibroblasts [J.A. Casas, et al., Ann. Rhem. Dis., Vol. 46, p. 763 (1987)], such as colchicine, which slows collagen secretion into the extracellular matrix [D. Kershenobich, et al., N. Engl. J. Med., Vol. 318, p. 1709 (1988)]. Other drugs act as inhibitors of key collagen metabolism enzymes [K. Karvonen, et al., J. Biol Chem., Vol. 265, p. 8414 (1990); C.J. Cunliffe,

et al., J. Med. Chem., Vol. 35, p.2652 (1992)]. However, none of these inhibitors have specific effects for the metabolism and deposition of specific types of collagen. Also, these drugs may interfere with the biosynthesis of other vital collagenous molecules, such as Clq in the classical complement pathway, acetylcholine esterase of the neuro-muscular junction endplate, conglutinin and pulmonary surfactant apoprotein. Such interference and lack of specificity could have potentially serious adverse effects.

Other drugs which can inhibit collagen synthesis, such as nifedipine and phenytoin, inhibit synthesis of other proteins as well, thereby non-specifically blocking the collagen biosynthetic pathway [T. Salo, et al., J. Oral Pathol. Med, Vol. 19, p. 404 (1990)]. Again, the lack of specificity significantly reduces the clinical use of these drugs, because the non-specific inhibition of protein synthesis can result in adverse side-effects when the drug is administered to the patient.

Indeed, clinically available anti-fibrotic drugs, including the collagen cross-linking inhibitors such as β-amino-propionitrile discussed previously, are also non-specific. Unfortunately, the lack of specificity of these collagen cross-linking inhibitors ultimately results in severe side effects after prolonged use. These side effects include lathritic syndrome, as well as disrupted elastogenesis. The latter side effect is a result of the disruption of cross-linking of elastin, another fibrous connective tissue protein. In addition, the collagen cross-linking inhibitory effect of these drugs is secondary, so that collagen must first be overproduced before degradation by collagenase. Thus, a type-specific inhibitor of the synthesis of collagen itself is clearly required.

Such a type-specific collagen synthesis inhibitor is disclosed in U.S. Patent No. 5,449,678 for the treatment of certain fibrotic conditions such as scleroderma and Graft Versus Host Disease. Both of these conditions are associated with excessive collagen deposition, which can be inhibited by Halofuginone. This specific inhibitor is a composition with a pharmaceutically effective amount of a pharmaceutically active compound of a formula:

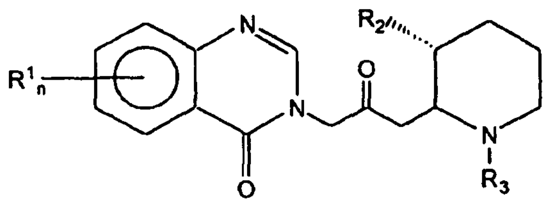

wherein: n = 1 or 2

$R_1$ is a member of the group consisting of hydrogen, halogen, nitro, benzo, lower alkyl, phenyl and lower alkoxy;
$R_2$ is a member of the group consisting of hydroxy, acetoxy and lower alkoxy; and
$R_3$ is a member of the group consisting of hydrogen and lower alkenoxy-carbonyl. Of this group of compounds, Halofuginone has been found to be particularly effective for such treatment.

International Patent Application No. WO- 96/ 06,616 further discloses that these compounds are able to effectively treat restenosis by preventing the proliferation of vascular smooth muscle cells. Restenosis is characterized by smooth muscle cell proliferation and extracellular matrix accumulation within the lumen of affected blood vessels in response to a vascular injury [Choi et al., Arch. Surg., Vol. 130, p. 257-261 (1995)]. One hallmark of such smooth muscle cell proliferation is a phenotypic alteration, from the normal contractile phenotype to a synthetic one. Type I collagen has been shown to support such a phenotypic alteration, which can be blocked by Halofuginone [Choi et al., Arch. Surg., Vol. 130, p.257-261 (1995); PCT Patent Application No. 96/ 06,616]. Thus, Halofuginone can prevent such abnormal redifferentiation of smooth muscle cells alter vascular injury by blocking the synthesis of type I collagen. Other in vitro studies show that Halofuginone can also inhibit the proliferation of 3T3 fibroblast cells [U.S. Patent No. 5,449,678].

International Patent Application No. WO- 97/ 06,805 discloses that compositions comprising quinazolinone of thé general formula I including halofuginone inhibit several enzymes participating in neovascularization, showing in in vitro studies that halofuginone inhibits proliferation of vascular endothelial cells and smooth muscle cells, as well as the synthesis of collagen and assembly of the sub-endothelial ECM, which are ail essential for microvessel tube formation.

However, the *in vitro* action of Halofuginone does not always predict its *in vivo* effects. For example, Halofuginone inhibits the synthesis of collagen type I in bone chrondrocytes *in vitro,* as demonstrated in U.S. Patent No. 5,449,678. However, chickens treated with Halofuginone were not reported to have an increased rate of bone breakage, indicating that the effect is not seen *in vivo.* Thus, the exact behavior of Halofuginone *in vivo* cannot always be predicted from *in vitro* studies.

Furthermore, the ability of Halofuginone or other related quinolinones to block or inhibit physiological processes related to tumor growth and progression is not known in the prior art. Although Halofuginone has been shown to have a specific inhibitory effect on the synthesis of type I collagen, such inhibition has not been previously shown to slow or

halt tumor progression, particularly *in vivo.* Indeed, the specific inhibition of the mechanism of angiogenesis alone has not been typically clinically employed for the treatment of malignancies.

Most clinically available approaches for the treatment of malignancies focus on cytotoxic therapies, such as chemotherapy or radiation treatments, in order to kill actively proliferating cells. Unfortunately, these therapies are highly toxic to non-cancer cells and cause severe side effects, such as bone marrow suppression, hair loss and gastrointestinal disturbances. In particular, these therapies are not able to specifically induce apoptosis in tumor cells, rather than simply killing all actively proliferating cells. However, the specific induction of apoptosis is clearly an important mechanism for selectively killing cancer cells without causing indiscriminate cell toxicity. Thus, clearly medications which cause apoptosis would be highly useful for the treatment of malignancies.

The treatment of malignancies, including the inhibition of primary tumor growth, tumor progression and metastasis, could thus potentially act by inhibiting or otherwise affecting a number of different mechanisms required for tumor growth and metastasis. These mechanisms, as described previously, include angiogenesis, deposition of collagen and the absence of normal apoptosis. Unfortunately, currently available treatments for malignancies focus on cytotoxicity, rather than upon the inhibition of any one of these mechanisms. Certainly, none of the currently available treatments are able to inhibit all of these mechanisms.

There is thus a widely recognized unmet medical need for an inhibitor of tumor growth, progression and metastasis, which is particularly effective *in vivo,* substantially without adversely affecting other physiological processes, and which is able to inhibit angiogenesis and

## SUMMARY OF THE INVENTION

Unexpectedly, it has been found, as described in the examples below, that Halofuginone can also slow or halt tumor progression *in vivo.* Without wishing to be limited to a particular hypothesis, Halofuginone may act through a number of different mechanisms. For example, Halofuginone may act by inhibiting angiogenesis, by blocking ECM deposition, by inhibiting collagenase type IV activity, by inducing apoptosis or by inhibiting H19 gene expression, or possibly through a combination of these mechanisms, although another mechanism or mechanisms could also be responsible. However, it is not desired to be limited to a single mechanism, nor is it necessary since the *in vivo* data presented below clearly demonstrate the efficacy of Halofuginone as an inhibitor of tumor progression *in vivo.*

According to an embodiment of the present invention, there is provided the use of Halofuginone and pharmaceutically acceptable salts thereof for preparing a medicament for treating a tumor selected from the group consisting of breast cancer, lung cancer, bladder cancer, rhabdomyosarcoma, angiosarcoma, adenocarcinoma of the colon, prostate or pancreas, squamous cell carcinoma of the cervix, ovarian cancer, malignant fibrous histiocytoma, skin cancer, astrocytoma, glioma and hepatocellularcarcinoma.

According to another embodiment of the present invention, there is provided the use of Halofuginone and pharmaceutically acceptable salts thereof for preparing a medicament for inhibiting cell proliferation enabled by a deposition of an extracellular matrix in a tumor selected from the group consisting of breast cancer, lung cancer, bladder cancer, rhabdomyosarcoma, angiosarcoma, adenocarcinoma of the colon, prostate or pancreas, squamous cell carcinoma of the cervix, ovarian cancer, malignant fibrous histiocytoma, skin cancer, astrocytoma, glioma and hepatocellularcarcinoma.

According to yet another embodiment of the present invention, there is provided the use of Halofuginone and pharmaceutically acceptable salts thereof for preparing a medicament for inhibiting metastasis in a tumor selected from the group consisting of breast cancer, lung cancer, bladder cancer, rhabdomyosarcoma, angiosarcoma, adenocarcinoma of the colon, prostate or pancreas, squamous cell carcinoma of the cervix, ovarian cancer, malignant fibrous histiocytoma, skin cancer, astrocytoma, glioma and hepatocellularcarcinoma.

More preferably, said breast cancer is infiltrating duct carcinoma of the breast. Alternatively and more preferably, said bladder cancer is bladder carcinoma. Also alternatively and more preferably, said skin cancer is malignant melanoma.

For all of the above embodiments, preferably said compound is Halofuginone and pharmaceutically acceptable salts thereof.

Hereinafter, the term "Halofuginone" is defined as a compound having a formula:

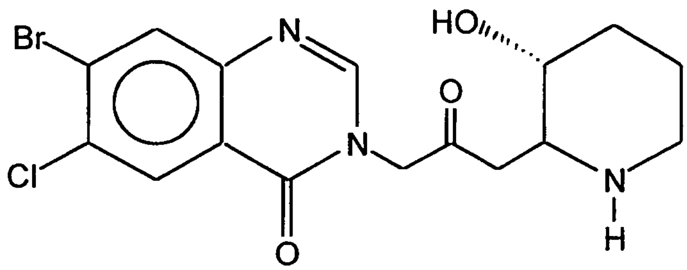

and pharmaceutically acceptable salts thereof. The composition preferably includes a pharmaceutically acceptable carrier for the compound.

Preferably, all of the compounds referred to hereinabove can be either the compound itself as described by the formula, and/or pharmaceutically acceptable salts thereof.

BRIEF DESCRIPTION OF THE DRAWINGS

The invention is herein described, by way of example only, with reference to the accompanying drawings, wherein:

FIGS. 1A-1L illustrate the inhibition of *in vivo* tumor growth, of five different tumors in seven different models of cancer in mice or rats, by Halofuginone;
FIGS. 2A and 2B illustrate the effect of Halofuginone on $^3$H-thymidine incorporation and proliferation of human leiomyosarcoma tumor cells;
FIG. 3 illustrates the dose-dependent inhibition of type IV collagenase activity in T50 bladder carcinoma cell cultures in the presence of Halofuginone;
FIG. 4 illustrates the inhibition of the expression of the H19 gene in the RT112 and 5376 bladder carcinoma cell lines by Halofuginone;
FIGS. 5A and 5B illustrate the induction of apoptosis in sections derived from the periphery of bladder tumors after administration of Halofuginone;
FIGS. 6A-6D show the induction of apoptosis in sections taken from the center of tumors after administration of Halofuginone;
FIGS. 7A-7D illustrate the inhibition of tumor cell proliferation by Halofuginone;
FIGS. 8A and 8B show the effect of Halofuginone on $^3$H-thymidine incorporation into bovine aortic endothelial cells maintained in culture, in the absence or the presence of bovine fibroblast growth factor (bFGF);
FIGS. 9A and 9B illustrate the inhibitory effect of Halofuginone on the organization of bovine aortic endothelial cells into capillary-like networks;
FIGS. 10A and 10B pictorially illustrate the inhibitory effect of Halofuginone on microvessel formation from rat aortic rings embedded in type I collagen gel;
FIG. 11 is a dose-response curve of the inhibitory effect of Halofuginone on microvessel formation, using the collagen Type I embedded rat aortic rings of Figures 10A and 10B;
FIG. 12 demonstrates the reversibility of the inhibitory effect of Halofuginone;
FIG. 13 shows the effect of Halofuginone on sulfate incorporation into the subendothelial ECM with bovine corneal endothelial cells;
FIGS. 14A-14D compare the effect of Halofuginone on incorporation of sulfate, proline, lysine and glycine into the ECM by bovine corneal endothelial cells;
FIGS. 15A-15D illustrate the effect of Halofuginone on sulfate and glycine incorporation into the ECM of rat mesengial cells;
FIGS. 16A-16F illustrate the inhibitory effect of Halofuginone on *in vivo* neovascularization in the eyes of mice;
FIG. 17 shows a Northern blot with the effect of Halofuginone on Integrin $\alpha_v$ chain expression; and
FIG. 18 shows the effect of Halofuginone on $\beta$ subunit expression as determined by RT-PCR.

BRIEF DESCRIPTION OF THE INVENTION

Halofuginone, and by extension the related quinazolinone derivatives described and claimed in U.S. Patent 3,320,124, inhibit tumor growth, progression and metastasis through a number of distinct inhibitory mechanisms, hereinafter termed "panstasis". These mechanisms include, but are not limited to, the inhibition of angiogenesis, the prevention of ECM deposition, the inhibition of collagenase type IV activity, the inhibition of integrin expression, the induction of apoptosis and the inhibition of H19 gene expression.

With regard to specific aspects of these mechanisms, angiogenesis and ECM deposition have been previously described. Collagenase type IV is a pivotal metalloprotease enzyme involved in metastasis and cell invasion. Apoptosis is programmed cell death which, as noted previously, is blocked in malignant cells, which are therefore also described as "immortal". The H19 gene is a tumor-marker gene associated with the early stages of bladder carcinoma. More specifically, the H19 gene is a developmentally regulated gene whose expression peaks during fetal development when tissue differentiation is occurring. Chromosomal abnormalities within the region containing H19 are associated with early stages of malignancies such as Wilms' tumor, adrenocortical carcinoma, hepatoblastoma, rhabdomyosarcoma, lung tumors, trophoblastic tumors and bladder carcinoma [B. Tycko, Am. J. Path., Vol. 144, p. 431-439, 1994; de Groot, N. et al., Trophoblast Res., Vol. 8, p. 2285-2302, 1994; Rachmilewitz, J. et al., Oncogene, Vol. 11, p. 863-870, 1995].

The importance of panstasis is that the mechanism of action of Halofuginone includes many different cytostatic activities, without being actively cytotoxic. Such cytostatic activities are less likely to induce drug resistance and are also less likely to produce adverse side effects by affecting non-cancer cells. Thus, Halofuginone can be differentiated from currently available therapies for cancer which are actively cytotoxic.

## DESCRIPTION OF PREFERRED EMBODIMENTS

Unexpectedly, it has been found, as described in the examples below, that Halofuginone can also slow or halt tumor progression *in vivo.* Irrespective of the specific mechanism, the data presented below clearly demonstrate the efficacy of Halofuginone *in vivo* at inhibiting tumor progression.

Such a finding is unexpected for three reasons. First, the behavior of Halofuginone *in vitro* does not exactly correspond to its behavior *in vivo.* This can be demonstrated by the differential effect of Halofuginone observed with bone chondrocytes *in vivo* and *in vitro.* Halofuginone inhibits the synthesis of collagen type I in chrondrocytes *in vitro,* as demonstrated in U.S. Patent No. 5,449,678. However, chickens treated with Halofuginone were not reported to have an increased rate of bone breakage, indicating that the effect is not seen *in vivo.* Thus, the exact behavior of Halofuginone *in vivo* cannot always be predicted from *in vitro* studies.

Second, the only previously known examples of the inhibition of cell proliferation by Halofuginone involved either smooth muscle cells which had become phenotypically altered in response to a vascular injury, or 3T3 fibroblasts [PCT Application No. 96/06616 and Choi et al., Arch. Surg., Vol. 130, p. 257-261 (1995)]. These cells simply proliferated without organization. By contrast, angiogenesis involves the formation of highly organized vascular structures. Thus, the finding that Halofuginone can inhibit such angiogenesis is both novel and non-obvious.

Furthermore, the examples given below clearly demonstrate that Halofuginone is also effective in the inhibition of cell proliferation enabled by the deposition of an extracellular matrix, *in vivo* as well as *in vitro.* Specific inhibition of collagen deposition has never been demonstrated before, particularly *in vivo.*

In addition, the data presented herein also demonstrate that Halofuginone is an effective inhibitor of type IV collagenase activity, of tumor-marker gene expression and of the inhibition of integrin expression. The data also demonstrate that Halofuginone is able to induce apoptosis of tumor cells. These effects of Halofuginone have not been demonstrated before.

Thus, nothing in the prior art taught or suggested that Halofuginone would be useful in the treatment of malignancies *in vivo.* Furthermore, the ability of Halofuginone, and related compounds, to slow or halt tumor progression, to inhibit cell proliferation enabled by the deposition of an extracellular matrix, to inhibit type IV collagenase activity, to inhibit integrin expression, and to induce apoptosis is both novel and non-obvious. The demonstration of such an ability *in vivo* is particularly unexpected, given the differential responses seen *in vitro* and *in vivo* to Halofuginone.

While the invention will now be described in connection with certain preferred embodiments in the following figures and examples so that aspects thereof may be more fully understood and appreciated, the invention is not intended to be limited to these particular embodiments. On the contrary, all alternatives, modifications and equivalents are included as within the scope of the invention as defined by the appended claims. Thus, the following figures and examples which include preferred embodiments will serve to illustrate the practice of this invention, it being understood that the particulars shown are by way of example and for purposes of illustrative discussion of preferred embodiments of the present invention only, and are presented in the cause of providing what is believed to be the most useful and readily understood description of formulation procedures as well as of the principles and conceptual aspects of the invention.

The present invention may be more readily understood with reference to the following illustrative examples and figures. It should be noted that although reference is made exclusively to Halofuginone, it is believed that the other quinazolinone derivatives described and claimed in U.S. Patent 3,320,124, the teachings of which are incorporated herein by reference, have similar properties.

Example 1

Inhibition of *in vivo* Tumor Growth in

Mice or Rats by Halofuginone

The inhibition of growth of five different types of tumors of mouse, rat and human origin was examined *in vivo* in three different strains of mice, C3H, CD1-nu athymic and C57BL/6 mice, and in the Fischer strain of rats. T50 bladder carcinoma was administered to C3H mice. In addition, bladder carcinoma was induced *in situ* in C3H mice by the addition of N-Butyl-N-4 hydroxybutyl nitrosamine to the drinking water of these mice. C57BL/6 mice were inoculated with EHS sarcoma cells. Nude mice were inoculated with MDA435, which is an aggressive variant of MCF-7 breast carcinoma; or melanoma. Fischer rats were stereotactically injected in the brain with a suspension of $1 * 10^5$ malignant fibrous histocytoma cells. Unexpectedly, Halofuginone was shown to have a significant inhibitory effect on tumor growth and progression in these *in vivo* models.

Inhibition of T50 Bladder Carcinoma by Halofuginone

For the first experiment, in which C3H mice were inoculated with T50 bladder carcinoma cells, C3H mice were divided into two groups of 6 mice each. The experimental group received a diet containing either 10 mg/kg or 5 mg/kg of Halofuginone 3 days prior to the injection of T50 bladder carcinoma cells and during 2 weeks after. Cultured T50 cells, a more aggressive variant of the chemically induced MBT2 mouse bladder carcinoma, were dissociated with trypsin/EDTA into a single cell suspension ($10^6$ cells/ml) in growth medium and inoculated s.c. in two sites on the dorsa of mice. The right side received $0.4 * 10^5$ cells, and the left side received $2 * 10^5$ cells. The tumor size was estimated by measurement of tumor length in two directions, using the formula $V = LW^2/2$, where V is volume, L is length and W is width. At the end of the experiment at day 17, the mice were weighed, the tumors were excised and a sample of the tumor tissue was fixed and processed for histological examination. The quantitative results of the experiment are shown in Figures 1A and 1B, and in Table 1 for the dose of 5 mg/kg. A photograph of representative bladder carcinoma bearing mice which were untreated (top) or treated (bottom) with Halofuginone is shown in Figure 1I. Representative tumors are shown in Figure 1C for the doses of 5 mg/kg and 10 mg/kg. Table 2 shows a comparison between the dose of 5 mg/kg and the dose of 10 mg/kg of Halofuginone.

T50 tumor size in C3H mice which were fed with 5 mg/kg Halofuginone was significantly reduced, by about 70-80%, as compared to control mice maintained on a normal diet, as shown in Figures 1A and 1B. The anti-tumor effect of Halofuginone was observed both at the high tumor cell dose, with a volume of $5.0 \pm 3.07$ cm$^3$ for control mice and $1.0 \pm 0.92$ cm$^3$ for Halofuginone containing diet, and the low tumor cell dose, with a volume of $1.63 \pm 0.98$ cm$^3$ for control mice and $0.29 \pm 0.28$ cm$^3$ for Halofuginone fed mice. Furthermore, the overall weight of the Halofuginone treated C3H mice was lower than the weight of the untreated mice, $24 \pm 3.1$ g and $40 \pm 3.8$ g, respectively, due to the lower tumor burden. Data for all mice for 5 mg/kg are shown in Table 1.

Table 1. Effect of Halofuginone on T50 Tumor Size (cm$^3$)

| | - Halofuginone | | + Halofuginone | |
|---|---|---|---|---|
| Sample No. | $0.4 * 10^5$ cells | $2*10^5$ cells | $0.4 * 10^5$ cells | $2*10^5$ cells |
| 1 | 2.6 | 4.3 | 0.036 | 0.7 |
| 2 | 2.7 | 10.1 | 0.064 | 0.1 |
| 3 | 0.4 | 6.1 | 0.150 | 1.1 |
| 4 | 1.0 | 6.6 | 0.730 | 2.0 |
| 5 | 0.9 | 0.7 | 0.510 | 1.1 |
| 6 | 2.2 | 2.2 | NA | NA |

There was a greater inhibitory effect when Halofuginone was administered at a dose of 10 mg/kg of the diet, as shown in Table 2 and Figure 1C.

| Table 2. Effect of Halofuginone on T50 Tumor Size ($cm^3$ Mean +/- SD) | | | | | |
|---|---|---|---|---|---|
| -Halofuginone (control) | | +Halofuginone (10 mg/kg) | | +Halofuginone (5 mg/kg) | |
| $0.4 * 10^5$ cells | $2 * 10^5$ cells | $0.4 * 10^5$ cells | $2 * 10^5$ cells | $0.4 * 10^5$ cells | $2 * 10^5$ cells |
| 1.430 ±0.705 | 2.620 ± 0.344 | 0.019 ± 0.016 | 0.244 ±0.47 | 0.843 ± 0.346 | 1.733 ± 0.720 |

Similar results were obtained when the tumors were excised and weighed. These results clearly illustrate the dose-dependence of the effect of Halofuginone against malignancies.

Halofuginone was then administered after the inoculation of the primary T50 bladder carcinoma tumor, to determine if Halofuginone still is able to inhibit the growth and progression of the tumor. For this purpose, 1 x $10^5$ T50 bladder carcinoma cells were injected s.c. (subcutaneously) and the mice were kept on a normal diet until the tumor appeared and reached a size of about 0.3 cm in diameter, which required about 8-10 days. The mice were then fed with a diet containing 5 mg/kg Halofuginone and the tumor size was measured on days 18 and 24 after tumor inoculation. As demonstrated in Figures 1D and 1E, tumor growth was suppressed by Halofuginone even when given 8-10 days after injection of the tumor cells. In fact, the inhibition of tumor growth in this group was similar to that obtained when Halofuginone was given 2 days prior to the tumor cell inoculation (0.99±0.55 $cm^3$ and 0.59±0.43 $cm^3$, respectively, n=8). The tumor size in the untreated group was 4.78±1.87 $cm^3$ (Figure 1E).

In addition, Halofuginone was administered i.p. in the amount of 1 μg per mouse per day, starting on the day of tumor cell injection. A profound inhibition of tumor growth was obtained on day 18 (0.55±0.28 $cm^3$ vs. 2.09±0.86 $cm^3$, in the treated vs. control mice, n=8) (Figure 1D) while only about 50% reduction in tumor size was observed on day 24 (Figure 1E). The experiment was terminated on day 27 after the tumor cell inoculation. At this time, only 25% of the control mice remained alive as compared to 63% and 71% of the mice that were treated with Halofuginone given either orally or i.p., respectively. The survival effect of Halofuginone given in the diet starting after the appearance of the tumor was less pronounced (only 38% of the mice remained viable).

Inhibition *of in situ* Bladder Cancer by Halofuginone

In the second experiment, in which bladder carcinoma was induced *in situ* in C3H mice, the C3H mice were divided into two groups of six mice each. Both groups received 0.05% BHBN (N-butyl-N-4 hydroxybutyl nitrosamine) through their drinking water to induce the carcinoma, thereby resembling the development of bladder cancer in humans. The experimental group of mice received a diet containing 5 mg/kg Halofuginone, while the control group (n=6) received 0.05% BHBN and were fed with a normal diet. After 26 weeks, the mice were weighed, the tumors were excised and a sample of the tumor tissue was fixed and processed for histological examination. The results of the experiment are shown in Figures 1F and 1G.

The difference in the size of the tumors is demonstrated in the histological sections stained with hematoxyline and eosine, and presented in Figures 1Fa (control mice) and 1Fb (Halofuginone treated mice). In the control group, all the characteristic stages of bladder tumor progression (dysplasia, carcinoma *in situ,* invasive carcinoma) were observed in all the mice, with massive invasion of tumor cells through the epithelial layer, basement membrane and bladder capsule (Figure 1G, A1 and A2). In contrast, in the Halofuginone treated mice only the first stages of tumorigenesis (i.e., dysplasia) were evident. Local cell invasion through the basement membrane was seen in only one case with no penetration of the bladder capsule (Figure 1G, B 1 and B2). The weight of mice which were treated with Halofuginone during the 26 weeks was 20-30% lower than the weight of mice from the control group, possibly due to the excess weight of the tumor itself in the control group.

Inhibition of EHS Tumors by Halofuginone

For the third experiment, C57BL/6 mice were inoculated with Engelbreth-Holm-Swarm (EHS) tumors. The experimental method was as follows. First, other animals bearing EHS tumors were sacrificed and the tumor tissue was excised and minced under aseptic conditions. The EHS tumor is characterized by a large amount of basement membrane components. A suspension of the tumor tissue in 0.2 ml of PBS was injected subcutaneously in the dorsal posterior region of C57BL/6 male mice. The mice were divided into two groups of 10 mice each. The animals of the experimental group were fed with a diet containing 5 mg/kg Halofuginone for 3 days prior to tumor injection and during 3 weeks after. The tumor size was estimated by measurement of tumor length in two directions, using the formula $V = LW^2/2$. On the final day of the experiment, i.e., 20 days after tumor injection, the mice were weighed, the tumors were excised and a sample of the tumor tissue was fixed and processed for histological examination. The quantitative results of the experiment are shown in Figure 1H and Table 3.

Tumor size in C57BL/6 mice which were fed Halofuginone was reduced by about 75% as compared to control mice, with volumes of 1.72 ± 1.85 $cm^3$ and 8.15 ± 4.88 $cm^3$, respectively, as shown in Figure 1H. The anti-tumor effect of Halofuginone was reflected by the nearly normal weight of Halofuginone-fed mice, 19.7 ± 0.47 g, as opposed to the approximately 1.6 fold higher weight of control mice, 33.3 ± 2.49 g, due to the tumor burden. Data for all mice are shown in Table 3.

Table 3. Effect of Halofuginone on EHS Tumor Size

| Sample No. | - Halofuginone | + Halofuginone |
|---|---|---|
| 1 | 12.6 | 1.3 |
| 2 | 5.8 | 1.2 |
| 3 | 3.2 | 1.3 |
| 4 | 12.6 | 1.7 |
| 5 | 4.9 | 0 |
| 6 | 6.8 | 0 |
| 7 | 4.9 | 2.2 |
| 8 | 18.9 | 0.04 |
| 9 | 2.8 | 6.5 |
| 10 | 9.0 | 3.0 |

Inhibition of Breast Carcinoma by Halofuginone

For the fourth experiment, highly aggressive human breast carcinoma cells (MDA435, an aggressive variant of the non-metastatic MCF-7 breast carcinoma cell line) were inoculated at $1 \times 10^6$ tumor cells per mouse s.c. to nude mice. The study group (n=4) received 5 mg/kg Halofuginone in the diet while the control group (n=4) was maintained on a regular diet. Visible tumors appeared within 4-5 weeks, the mice were sacrificed after 15 weeks and the tumors were excised and weighted. Despite a high variability within each group, there was a significant ($p < 0.024$) reduction in tumor weight in the Halofuginone treated mice (0.14±0.11g) vs. the untreated control mice (0.65±0.45 g).

In another experiment, Halofuginone was administered i.p in an amount of 1 μg per mouse, every other day. In this experiment CD1-nu athymic female mice were injected with the estrogen dependent non-metastatic parental MCF-7 human breast carcinoma cell line. The cells were implanted in the mammary pads of the mice at a concentration of $1 \times 10^7$ cells/0.5 ml. All animals were implanted with slow release pellets of 17 β-estradiol (0.72 mg/pellet, 60-day release; Innovation Research of America). When the tumors of the control mice reached an average size of about 1 $cm^3$ (about 10 weeks after implantation), the tumors were excised (Figure 1J). The appearance of control mice and mice treated with oral Halofuginone is presented as a photograph in Figure 1K.

Inhibition of Melanoma Tumors by Halofuginone

For the fifth experiment, nude mice were inoculated with melanoma tumors. The experimental method was as follows. First, a suspension of $10^6$ tumor cells was injected subcutaneously in the dorsal posterior region of nude mice. The mice were divided into two groups of five mice each. The animals of the experimental group were injected i.p. with 1 μg of Halofuginone per mouse every other day, starting one week before inoculation with the tumor and for the following four weeks after inoculation. Control mice were injected with saline. The results are shown as a photograph in Figure 1L.

Black superficial big melanoma tumors were observed in all mice in the control group (-H). In the group treated with Halofuginone (+H), all mice had significantly smaller tumors which had not reached the black superficial melanoma tumor stage. Thus, significant inhibition of tumor growth was achieved in mice treated with Halofuginone as compared with control mice, which did not receive Halofuginone.

Inhibition of Brain Tumors by Halofuginone

For the sixth experiment, Fischer rats were inoculated with brain tumors. Since the molecular weight of Halofuginone is low, and Halofuginone is hydrophobic, the molecule was expected to be able to penetrate the blood-brain barrier (BBB), an expectation which is supported by the experimental results. Despite the concern that the BBB impairs

chemotherapy entry, this barrier is selectively disrupted at the site of malignant lesion, and therefore, the tumor will receive higher doses of systemically administered chemotherapy than the adjacent normal brain. However, in both animal and human studies, most agents are more effective against systemic tumors than against the metastases to the brain. This is probably related to an inadequate and slow influx of compounds into brain tumors, an outcome of reduced blood flow and increased interstitial pressure resulting from edema formation and increased intracranial pressure.

The brain tumor tested was an unselected rat tumor line of a methylcholantrene-induced malignant fibrous histocytoma, maintained in syngeneic Fischer rats by serial subcutaneous transplantations. For stereotactic brain inoculation, the tumor cell suspension was injected into the right cerebral hemisphere of adult female Fischer rats (weighing 180-200 g), using a small animal stereotactic apparatus. With bregma as zero reference point, the stereotactic coordinates were: P=3.5; L=2; H=-4 mm. The head was held in the horizontal position and the volume of injection was 2 μl. The number of injected tumor cells per rat was $10^5$.

Ten rats received a daily intraperitoneal injection of Halofuginone (16 μg/rat) starting two days prior to inoculation of the tumor cells. Starting on day 4 post inoculation, 5 mg/kg Halofuginone was administered in the diet instead of i.p. Control animals received i.p. injections of saline and normal diet. On days 9, 11,13 and 15 after stereotactic injection of the tumor cells, two of the animals in each group were sacrificed by an overdose of pentobarbital. The visible tumor mass was weighed and the brain was rapidly removed, placed in a cutting chamber and cut into 3-mm thick coronal sections. The visible tumor mass was weighed and measured in three dimensions. The tumors of two animals of each group were processed for pathological examination.

A most pronounced decrease in tumor size was seen in each the Halofuginone treated rats as compared to the untreated counterpart animals. In fact, in 4 out of 8 Halofuginone treated rats there was almost no visible tumor (1-8 $mm^3$) as compared to all ten control animals having tumors ranging in size from 80 $mm^3$ (day 9) to 260 $mm^3$ (day 13) (Table 4). The anti tumoral effect of Halofuginone was also reflected by death of the two rats left untreated for 15 days as compared to the treated rats which remained viable.

Table 4. Effect of Halofuginone on Tumor Volume ($mm^3$)

| Day | -Halofuginone | +Halofuginone |
|---|---|---|
| 9 | 90 | 1 |
| | 81 | 42 |
| 11 | 154 | 1 |
| | 210 | 81 |
| 13 | 260 | 8 |
| | 260 | 375 |
| 15 | died | 0 |
| | died | 315 |

These six separate experiments, performed on both rats and mice, show that Halofuginone is capable of inhibiting tumor growth and progression *in vivo* for a wide variety of malignancies, including bladder carcinoma, breast cancer, melanoma, EHS sarcoma and brain tumors. The ability of Halofuginone to inhibit the growth and progression of brain tumors is particularly exciting, since the blood-brain barrier limits the penetration of many anti-neoplastic drugs into the brain. The administration of Halofuginone for relatively long periods of time, such as 26 weeks for the *in situ* bladder cancer experiment in mice, did not cause increased morbidity. Thus, Halofuginone represents an effective and safe treatment for a wide variety of cancers, a result which was not taught or suggested by the prior art.

Example 2

Effect of Halofuginone on Proliferation of Human Leiomyosarcoma Tumor Cells

The effect of Halofuginone on proliferation of human leiomyosarcoma tumor cells was investigated. Leiomyosarcoma tumors have abundant extracellular matrix and are also well vascularized [A. Ferenczy, et al., Cancer, No. 28, pp. 1004-1018 (1971)]. Their growth is thought to be dependent on growth factors (i.e., bFGF, HB-EGF) produced by normal and malignant myometrial cells [A. Zhang, et al., Endocrinology, No. 134, pp. 1089-1094 (1994); R.S. Mangrulker, et al, Biology of Reproduction, No. 53, pp. 636-646 (1995)] and locally embedded in the surrounding ECM [I. Vlodavsky, et al., Basement Membranes: Cellular and Molecular Aspects, D.H. Rohrbach and R. Timpl, Eds., Academic

Press, Inc., Orlando, Florida, U.S.A., pp. 327-343 (1993)].

Samples of human leiomyosarcoma tumors were obtained from women undergoing surgical hysterectomy, as described [R.S. Mangrulker, *et al., ibid.]*. Minced tissue was placed into 20 ml cold homogenization buffer: 1 M NaCl, 10 mM Tris (pH 7.4), 1 mM EDTA, 1 mM benzamidine, 0.1 % CHAPS, 0.01% Aprotinin (Sigma), 10 μg/ml leupeptin, 1 mM AEBSF [R.S. Mangrulker, *et al., ibid*.]. Samples were homogenized for 2 min and centrifuged at 12000 x g for 60 min at 4˚C. The supernatants were diluted 1:5 with 10 mM Tris (pH 7.4) to a final volume of 100 ml and filtered with 0.45 μm nylon filters. Cells were plated and maintained in DMEM plus 10% calf serum. The cells remained viable over a number of passages but were used at passages 2 or 3.

For proliferation assays, cells were plated in 96-well plates at 10,000 cells/well in 200 μl medium (DMEM with 4.5 g/L glucose, 10% calf serum, 1% glutamine, 1% penicillin/streptomycin) and incubated 2 days until confluent. 24 h after seeding, increasing concentrations of Halofuginone (10-100 ng/ml) were added. The medium was changed to DMEM with 0.5% calf serum, 1 μM insulin and 5 μM transferrin. After 24 h, the samples (5-10μl) were added and after an additional 24h, [$^3$H]thymidine (1 μCi/well) was added to each well. After 36-48 h incubation, the cells were fixed with methanol, and the DNA was precipitated with 5% trichloroacetic acid. The cells were lysed with 150 μl/well of 0.3 N NaOH, transferred to scintillation vials, and counted on a β-counter. As demonstrated in Figure 2A, 60-70% inhibition of [$^3$H]thymidine incorporation was obtained at 2.5 ng/ml Halofuginone.

The effect of Halofuginone on proliferation of HB-EGF (Heparin-Binding Epidermal Growth Factor) and serum-stimulated leiomyosarcoma cells was then examined. The leiomyosarcoma tumor cells were growth-arrested by 48 h incubation in medium containing 0.5% FCS. The cells were then exposed (24 h) to either 10% FCS or 10 ng/ml HB-EGF in the absence or presence of 10 ng/ml Halofuginone. [$^3$H]thymidine was then added and DNA synthesis measured 36 h later. A complete inhibition of cell proliferation induced by both serum or HB-EGF was observed in the presence of Halofuginone, as shown in Figure 2B.

## Example 3

## Inhibition of Collagen Type I Gene Expression by Halofuginone

Myometrial and leiomyosarcoma cells were taken from the same patient and were plated into 10 cm plates in DMEM supplemented with 10% FCS. When the cells reached 80% confluence, the medium was replaced by serum free DMEM plus 0.1% BSA for 48 hours, washed and exposed to increasing concentrations of Halofuginone in the same medium for about 48 hours at about 37 ˚C. The cells were then harvested and subjected to RNA extraction and Northern blot analysis for collagen type I gene expression. Halofuginone inhibited collagen type I gene expression (products at 5.4 and 4.8 kb) in a dose-dependent manner.

## Example 4

## Inhibition of Type IV Collagenase Activity by Halofuginone *in vitro*

Tumor cells secrete enzymes which digest the ECM, enabling the cells to burrow through neighboring tissue and to invade other tissues. Numerous studies have linked matrix metalloproteases (MMP), especially type IV collagenase, to the process of tumor invasion and metastasis. Type IV collagenase appears as two 72 and 92 kDa proteins encoded by a unique mRNA.

As demonstrated in Figure 3, a profound inhibition of the activity of MMP2 (72 kDa type IV collagenase) in T50 bladder carcinoma cell cultures was exerted in the presence of 25 ng/ml Halofuginone, while an almost complete inhibition was obtained at 100 ng/ml Halofuginone. Sub-confluent cell cultures were incubated for 6 - 24 h in serum-free DMEM. The collagenolytic activity was determined on a gelatin impregnated (1 mg/ml. Difco, Detroit, MI) SDS-PAGE 8% gel. Briefly, culture media samples were separated on the substrate impregnated gels under non reducing conditions, followed by 30 min incubation in 2.5% Triton X-100 (BDH, England). The gels were then incubated for 16 h at 37˚C in 50 mM Tris, 0.2 M NaCl, 5 mM $CaCl_2$, 0.02% Brij 35 (weight/volume) at pH 7.5. At the end of incubation period, the gels were stained with 0.5% Coomassie G250 (Bio-Rad Richmond CA) in methanol/acetic acid/$H_2O$ (30:10:60). The intensity of the various bands was determined on a computerized densitometer (Molecular Dynamics type 300A).

Halofuginone was also found to inhibit cell invasion through matrigel ECM, using the Boyden chamber invasion assay (data not shown). Such inhibition supports the inclusion of type IV collagenase inhibition as part of the panstasis mechanism, in which Halofuginone inhibits tumor growth, progression and metastasis through cytostatic activities, as described previously.

Example 5

Inhibition of Tumor-Marker Gene Expression by Halofuginone *in vitro*

The H19 gene is a developmentally regulated gene whose expression peaks during fetal development when tissue differentiation is occurring. The H19 gene is parentally imprinted, expressed only by the maternal allele. H19 is also a tumor-marker gene, associated with early stages of malignancies such as Wilms' tumor, adrenocortical carcinoma, hepatoblastoma, rhabdomyosarcoma, lung tumors, trophoblastic tumors and bladder carcinoma. The experimental method was as follows.

RT112 and 5376 human bladder carcinoma cell lines were cultured in the absence and presence of Halofuginone (130 ng/ml, added 24 h or 72 h after seeding), and the expression of the H19 gene was evaluated by Northern blot analysis (Nagler, A. et al., Arterioscler. Thromb. Vasc. Biol., Vol. 17, p. 194-202, 1997). Exposure to Halofuginone resulted in a substantial reduction in the expression of the H19 gene in the RT112 and 5376 bladder carcinoma cell lines which were tested, as shown in Figure 4. This result corroborates the *in vivo* studies of Example 1 on the anti-tumorigenic effect of Halofuginone in bladder carcinoma.

Such inhibition also supports the inclusion of the inhibition of H 19 gene expression as part of the panstasis mechanism, in which Halofuginone inhibits tumor growth, progression and metastasis through cytostatic activities, as described previously.

Example 6

Induction of Apoptosis by Halofuginone *in vivo*

Apoptosis, or programmed cell death, is determined by a mechanism within normal cells which is eliminated by malignant cells. Malignant cells are often described as "immortal" because of the absence of apoptosis. Halofuginone was shown to induce apoptosis of tumors *in vivo.* The experimental method was as follows.

Histological sections were taken from the periphery and center areas of bladder carcinoma tumors induced *in situ* in mice from Example 1. These sections were then subjected to *in situ* labeling of fragmented DNA using the terminal deoxynucleotidyl transferase (TdT) labeling technique [Holmgren, L. et al., Nat. Med., Vol. 1, p. 149-153, 1995]. As demonstrated in Figure 5B, a much higher number of apoptotic cells was observed in sections derived from bladder tumors of oral Halofuginone (10 mg/kg) treated C3H mice, as compared to tumors derived from untreated control mice (Figure 5A). A profound difference in the extent of staining was also seen in tissue sections derived from the center of the tumor undergoing necrosis. Figures 6A and 6C show the tumors of untreated mice, while Figures 6B and 6D show the tumors of Halofuginone treated mice. Apoptosis differs from necrosis in that apoptosis is characterized by single-cell death in the midst of living cells, as shown in Figure 5B. The increased apoptotic index observed in response to Halofuginone may be due to the above described anti-angiogenic effect, as observed with several recently developed anti-angiogenic agents.

Overall, an increased apoptotic index of about 10 fold magnitude was observed in tissue samples taken from mice to which Halofuginone was administered, in comparison to the control mice which did not receive Halofuginone. Such inhibition also supports the inclusion of the induction of apoptosis as part of the panstasis mechanism, in which Halofuginone inhibits tumor growth, progression and metastasis through cytostatic activities, as described previously.

Example 7

Inhibition of Cell Proliferation by Halofuginone

The anti cancer effect of Halofuginone may be attributed to an effect on i) collagen synthesis and matrix deposition (stromal support); ii) tumor angiogenesis; and iii) direct inhibition of tumor cell proliferation. The effect of Halofuginone on proliferation of T50 bladder carcinoma and other tumor cell lines maintained *in vitro* was therefore tested. The cells were seeded (2000 cells per 16 mm well of a 24-well plate) in DMEM containing 10% FCS. Increasing concentrations of Halofuginone were added 24 h afterwards and the cells were dissociated and counted in a Coulter counter at various days after seeding. The results were as follows.

Complete inhibition of T50 bladder carcinoma cells was obtained at 30 ng/ml Halofuginone with little or no effect at 25 and 10 ng/ml (Figure 7A). The proliferation of human melanoma (A375), prostate (PC3) and breast carcinoma (MDA 231, MDA 435) cells was not affected by Halofuginone up to a concentration of 25 ng/ml and was partially inhibited only in the presence of 100 ng/ml Halofuginone (Figures 7B and 7C). Likewise, there was only a small inhibitory effect of Halofuginone on human myeloid leukemic cells (HL60) and mouse B lymphoma cells (F32) (Figure 7D). The anti-tumoral effect of Halofuginone in the T50 bladder carcinoma may be due to the combined effect on matrix deposition,

angiogenesis and tumor cell proliferation.

Example 8

Halofuginone-induced inhibition of $^3$H-thymidine incorporation into vascular endothelial cells

Cultures of vascular endothelial cells were established from bovine aorta as previously described [D, Gospodarowicz, et al., Proc. Natl. Acad. Sci. U.S.A., Vol. 73, p. 4120 (1979)]. Bovine aortic endothelial cells were plated ($4x10^4$ cells/16 mm well) in DMEM (1 g glucose/liter) supplemented with 10% calf serum, 50 U/ml penicillin, and 50 μg/ml streptomycin at 37˚C in 10% $CO_2$ humidified incubators.

Four days after plating, the subconfluent cells were exposed to increasing concentrations of Halofuginone (25-500 ng/ml), in the absence or presence of 1 ng/ml bFGF. $^3$H-thymidine (1 μCi/well) was then added for an additional 48 hours, and DNA synthesis was assayed by measuring the radioactivity incorporated into trichloroacetic acid insoluble material [M. Benezra, et al., Cancer Res., Vol. 52, pp. 5656-5662 (1992); I. Vlodavsky, et al., Proc. Natl. Acad. Sci. U.S.A., Vol. 84, pp. 2292-2296 (1987)].

Figure 8A shows the results obtained in the absence of bFGF, while Figure 8B shows the results obtained in the presence of bFGF. As demonstrated in Figures 8A and 8B, 50% inhibition of $^3$H-thymidine incorporation was obtained at 100 ng/ml Halofuginone, regardless of whether or not bFGF ( 1 ng/ml) was added to the culture medium.

Example 9

Organization of Endothelial Cells into Capillary-like Networks

Halofuginone was found to prevent the organization of endothelial cells into a defined structure, and specifically inhibited the organization of these cells into capillary-like networks. Results are shown in Figures 9A and 9B.

Type I collagen was prepared from the tail tendons of adult Sprague-Dawley rats. Briefly, the collagen fibers were solubilized by a slow stirring for 48 h at 4˚C in a sterile, 1/1000 (v/v) acetic acid solution (300 ml for 1 g of collagen). The resulting solution was filtered through a sterile triple gauze and centrifuged at 16,000 g for 1 h at 4˚C. The supernatant was then extensively dialyzed against 1/10 DMEM and stored at 4˚C. The collagen matrix gel was obtained by simultaneously raising the pH and ionic strength of the collagen solution. For this purpose, 7 vol of collagen solution were quickly mixed with 1 vol of 10X Minimum Essential Medium and 2 vol sodium bicarbonate (0.15M) [R.F. Nicosa and A. Ottinetti, Lab. Invest., Vol. 63, pp. 115-122 (1990)].

Bovine aortic endothelial cells were seeded into 16 mm wells of a 24-well plate and allowed to grow for 24 h, to obtain a subconfluent monolayer. The culture medium was then removed and 0.4 ml of the cold collagen mixture described above were poured on top of the cell monolayer and allowed to polymerize for 10 min at 37˚C. Fresh medium (0.6 ml), containing bFGF (1 ng/ml) and heparin (1 μg/ml), with (Figure 9B) or without (Figure 9A) 0.1 μg/ml of Halofuginone, was added after the collagen had gelled. The reorganization of the endothelial cell monolayer was monitored and photographed with a Zeiss inverted phase contrast photomicroscope [R. Monesano, et al., J. Cell Biol., Vol. 97, pp. 1648-1652 (1983)].

Figure 9A illustrates the organization of endothelial cells into capillary-like networks. Such organization is inhibited by Halofuginone, as demonstrated by Figure 9B. Halofuginone completely inhibited the invasion of the endothelial cells into the collagen gel and their subsequent organization into a network of branching and anastomosing capillary-like tubes.

Example 10

Microvessel Formation

Halofuginone was shown to inhibit microvessel formation from rings of aortic tissues taken from rats. This effect was also shown to be reversible upon removal of Halofuginone. Results are shown in Figures 10A, 10B, 11 and 12.

Thoracic aortas were obtained from 1- to 2-month-old SD (Sprague-Dawley) rats sacrificed by decapitation [R.F. Nicosia and A. Ottinetti, Lab. Invest., Vol. 63, pp. 115-122 (1990)]. The aortas were immediately transferred to a Petri dish with PBS. The fibro-adipose tissue around the aorta was carefully removed under a dissecting microscope, and 1 mm-long aortic rings were sectioned and extensively rinsed in PBS.

Type I collagen solution (0.2 ml) was added to each 16-mm well and gellation was allowed for 15 min at 37˚C. Each aorta ring was transferred and positioned to the center of the gel and another 0.4 ml of the collagen solution was carefully poured on top of the ring. After the gel was formed, 0.4 ml of serum-free, endothelial growth medium, with or without 0.1 μg/ml Halofuginone, was added and the medium was changed every other day.

Figure 10A shows the culture at day 10, when the newly-formed branching microvessels were developed from the end of resection of the aorta, giving rise to loops and networks. Figure 10B shows the same culture treated with 0.1 μg/ml Halofuginone, replaced every other day. Under these conditions, single cells were migrating from the aortic ring toward the periphery, but failed to align into microvessel tubes.
Figure 11 shows this effect quantitatively, at increasing doses of Halofuginone. An almost complete inhibition of microvessel formation was obtained at 100 ng/ml Halofuginone. Complete inhibition was observed in the presence of 250 ng/ml Halofuginone. This effect was reversed upon removal of the drug on day 2, as shown in Figure 12. Such removal resulted in microvessel formation, similar to that seen with untreated aortic rings.

Example 11

Halofuginone Inhibition of Sulfate Incorporation into ECM of Cultured Endothelial Cells

Halofuginone was shown to have an inhibitory effect on the deposition of ECM (extracellular matrix components), as shown in Figure 13 and in other examples below.
Cultures of bovine corneal endothelial cells were established from steer eyes and maintained as previously described [D. Gospodarowicz, et al., Exp. Eye Res., No. 25, pp. 75-89 (1977)]. Cells were cultured at 37˚C in 10% $CO_2$ humidified incubators and the experiments were performed with early (3-8) cell passages.
For preparation of sulfate-labelled ECM (extra-cellular matrix), corneal endothelial cells were seeded into 4-well plates at a confluent density forming, within 4-6 h, a contact inhibited cell monolayer composed of closely apposed, and growth arrested cells. Under these conditions, the cells remained viable and retained their normal monolayer configuration and morphological appearance up to a concentration of 2 μg/ml Halofuginone. $Na_2[^{35}S]O_4$ (540-590 mCi/mmol) was added (40 μCi/ml) one and five days after seeding and the cultures were incubated without medium change. At various intervals after seeding, the subendothelial ECM was exposed by dissolving (5 min., room temperature) the cell layer with PBS containing 0.5% Triton X-100 and 20 mM $NH_4OH$ followed by four washes in PBS [I. Vlodavsky, et al., Cancer Res., Vol. 43, pp. 2704-2711 (1983); I. Vlodavsky, et al, Proc. Natl. Acad. Sci. USA, Vol. 84 pp. 2292-2296 (1987)]. To determine the total amount of sulfate labeled material, the ECM was digested with trypsin (25 μg/ml, 24 h, 37˚C) and the solubilized material counted in a β-counter.
Figure 13 shows the almost complete inhibition of sulfate incorporation by 1 μg/ml Halofuginone. 50% inhibition was obtained in the presence of 0.2 μg/ml of the drug (not shown).

Example 12

Inhibition of Incorporation of Sulfate, Proline, Lysine and Glycine into ECM of Bovine Corneal Endothelial Cells

Corneal endothelial cells were seeded at a confluent density and grown as described in Example 11 above. The cells were cultured with or without Halofuginone in the presence of either $Na_2{}^{35}SO_4$ (Figure 14A), $^3$H-proline (Figure 14B), $^{14}$C-lysine (Figure 14C) or $^{14}$C-glycine (Figure 14D). Eight days after seeding, the cell layer was dissolved substantially as described in Example 11 above. The underlying ECM was then either trypsinized to determine the effect of Halofuginone on incorporation of labeled material into total protein, substantially as described in Example 11 above, or subjected to sequential digestions with collagenase and trypsin to evaluate the effect of Halofuginone on both collagenase-digestible proteins (CDP) and non-collagenase digestible proteins (NCDP).
As Figures 14A-14D show, Halofuginone inhibited the incorporation of sulfate, proline, lysine and glycine into both CDP and NCDP, reflecting a profound inhibition of matrix deposition. The inhibitory effect of Halofuginone on deposition of ECM components other than collagen is most likely due to the involvement of collagen in the assembly of other constituents into the supramolecular structure of the ECM. Alternatively, Halofuginone may affect the synthesis of ECM components other than collagen, possibly through a common transcription factor or cytokine such as TGFβ, which affects the synthesis and deposition of several ECM components.

Example 13

Inhibition of Sulfate and Glycine Incorporation into Rat Mesengial Cell ECM

Rat mesengial cells were grown to confluency, 24 hours after seeding. The cells were then cultured with or without Halofuginone in the presence of either $Na_2{}^{35}SO_4$ (Figures 15A and 15B) or $^{14}$C-glycine (Figures 15C and 15D). Eight days after seeding, the cell layer was dissolved to expose the underlying ECM, washed and digested with collagenase to determine the effect of Halofuginone on CDP proteins, as shown in Figures 15A and 15C. The remaining material was digested with trypsin and subjected to β-scintillation counting to determine the effect of Halofuginone on

NCDP proteins, as shown in Figures 15B and 15D.

About 30% inhibition of sulfate incorporation was seen for CDP proteins, while about 70% inhibition was seen for NCDP proteins in the presence of 200 ng/ml Halofuginone. It should be noted that the inhibition of ECM deposition by Halofuginone was not due to its anti-proliferative activity since the drug was added to highly confluent, non-dividing cells. Since inorganic sulfate is incorporated primarily into sulfated glycosaminoglycans and not into collagen, it is conceivable that by inhibiting type I collagen synthesis, Halofuginone interferes with the asssembly of other ECM macromolecules, such as heparin sulfate proteoglycans, which are known to specifically interact with collagen to form ECM.

About 80% inhibition of glycine incorporation was seen for both CDP and NCDP proteins in the presence of 50 ng/ml Halofuginone. The inhibitory effect of Halofuginone on deposition of collagenase-digestible ECM proteins was more pronounced with glycine than with sulfate labeled matrix since unlike glycine, sulfate is incorporated primarily into glucosaminoglycans which are not degraded by collagenase. A profound inhibition of ECM deposition was supported by a microscopic examination of the denuded culture dishes, revealing a thin or non-existant layer of ECM produced in the presence of Halofuginone.

## Example 14

### Inhibitory Effect of Halofuginone on *In Vivo* Neovascularization

Halofuginone was shown to inhibit angiogenesis in an *in vivo* model. Such an inhibitory effect also demonstrates the ability of Halofuginone to inhibit cell proliferation which is enabled by the deposition of ECM components. Results are given in Figures 16A and 16B, and other examples below.

A murine corneal angiogenesis model was used to evaluate the inhibitory effect of Halofuginone in vivo. The angiogenic factor bFGF was applied into a corneal pocket of C57B1/6 mice, in a pellet made of a slow release polymer, and Halofuginone (2 μg/mouse/day) was administered i.p. for 5 consecutive days (Kenyon, B.M. et al., Invest. Ophthal. Visual Sci., Vol. 37, p. 1625-1632, 1996).

On post-operative day 5, mice were anesthetized with methoxyflurane, the eyes were proptosed, and the maximum vessel length (VL) of the neovascularization zone extending from the base of the limbal vascular plexus toward the pellet was measured with a linear reticule through the slit lamp. The contiguous circumferential zone of neovascularization (CH) was measured as clock hours with a 360 degree reticule. The eyes were photographed on day 5 and the slides were used to determine the area of neovascularization, in square mm (Kenyon, B.M. et al., Invest. Ophthal. Visual Sci., Vol. 37, p. 1625-1632, 1996).

The area of neovascularization was calculated according to the following formula.

$$A = \frac{(CH * 0.8) * 0.5 * VL * \pi}{2}$$

where A is area, CH is contiguous circumferential zone of neovascularization and VL is maximal vessel length.

Halofuginone was administered either in the food (5 mg/kg) or topical in the form of eye drops (100 and 200 ng/ml, applied three times per day). Control mice received regular diet. Each group contained 5 mice. On postoperative day 7 corneas were examined by slit lamp biomicroscopy to determine the neovascular response and photographed. Angiogenesis was measured in mm as the maximal vessel length (VL) of the neovascularization zone from the limbal vasculature and the pellet as was the contiguous circumferential zone of neovascularization (CH).

As demonstrated in Figure 16, neovascularization from the corneal limbus to the pellet occurred in the eyes of control mice (Figure 16A). A profound inhibition of the neovascular response to the pellet was observed in mice receiving oral Halofuginone (Figure 16B). The average area of neovascularization in the control group was 1.59$\pm$0.25 mm$^2$, as compared to 0.15$\pm$0.2 mm$^2$ in mice that received oral Halofuginone (Figure 16C).

As demonstrated in Figures 16C and 16D, topical application of Halofuginone 3 times a day yielded a significant inhibition of neovascularization both at 100 ng/ml (0.93$\pm$0.62 mm$^2$, p<0.001) and 200 ng/ml (0.56$\pm$0.6 mm$^2$; p<0.001; ~95% inhibition). The neovascularized zone measured on day 10 in the untreated group was 4.92$\pm$1.96 mm$^2$, as compared to 0.23$\pm$0.47 (~95% inhibition; p<0.001) in mice which received Halofuginone in the diet (5 mg/Kg) (Figure 16D).

The concentration of the topically applied Halofuginone was increased to 500 and 1000 ng/ml, given 3 times a day. Neovascularization was assessed on days 4, 7 and 10 post implantation of the bFGF pellets. Similar to the previous experiments, oral Halofuginone was more effective (~70% inhibition) than Halofuginone applied topically even at 1000 ng/ml (40-50% inhibition). Also, even at this high concentration of Halofuginone there was no local induration or irritation of the eye. Regression of the newly formed blood vessels was observed on day 10 (Figure 16E).

The anti-angiogenic effect of Halofuginone administered i.p (1 μg/mouse/day) was compared to the effect of the oral and topical modes of administration. Halofuginone (i.p, day 7) inhibited neovascularization from 1.75 $mm^2$ in the untreated group to 0.63 $mm^2$ ($p<0.05$), similar to the effect of oral Halofuginone (0.65 $mm^2$) in the same experiment. Halofuginone drops (500 ng/ml, x3 per day) were less effective (0.98 $mm^2$) (Figure 16F). Also, in one experiment the anti-angiogenic effect of Halofuginone was tested in C3H mice and the results were similar to those obtained with C57BL mice.

Thus, the results show that Halofuginone is a potent and non toxic anti-angiogenic compound, effective both when administered orally or i.p. Halofuginone was less effective when topically applied as eye drops, most probably from being washed away without achieving the desired constant concentration. Halofuginone appears to be the first compound described in the literature which exerts an anti-angiogenic effect when administered orally.

Example 15

Inhibition of Integrin Expression

Integrins have been shown to function *in vivo* in vasculogenesis and angiogenesis. Injection of neutralizing antibody against the ß1 subunit blocked the formation of an aortic lumen in quail embryos (Drake, C.J. et al., in vivo. Dev. Dyn. Vol. 193, p. 83-91, 1992). Cheresh and colleagues have provided evidence that $\alpha_v ß_3$ is required for blood vessel growth (Brooks, P.C. et al., Science Vol. 264, p. 569-571, 1994). An antibody (LM609) against the $\alpha_v ß_3$ integrin complex inhibited normal vessel growth and also FGF-2 stimulated or tumor-induced angiogenesis in the CAM assay, but did not disrupt preexisting vessels. The mechanism by which anti-$\alpha_v ß_3$ mAb disrupts angiogenesis appears to involve apoptosis. A single intravascular injection of a cyclic RGD peptide antagonist of $\alpha_v ß_3$ integrin or of the LM609 monoclonal antibody leads to the rapid regression of human tumors transplanted into the CAM. Tumor cells that fail to express the $\alpha_v$ gene and hence the $\alpha_v ß_3$ integrin, lose their adhesion capability and exhibit a significantly reduced tumorigenicity upon transplantation into athymic nude mice. Stable transfection of the $\alpha_v$ cDNA to these cells resulted in the full restoration of their tumorigenic potential (Felding-Habermann, B. et al, J. Clin. Invest., Vol. 89, p. 2018-2022, 1992). Furthermore, Halofuginone has been found to inhibit angiogenesis and tumor growth.

Therefore, the effect of Halofuginone was investigated on the expression of $\alpha_v$, $ß_3$ and $ß_5$ integrin subunits in the highly aggressive MDA 435 human breast carcinoma cell line. Cells were cultured in the absence (Figure 17, lanes A & B) or presence of increasing concentrations (10-400 ng/ml) of Halofuginone for 24 h (lane C: 400 ng/ml), 48 h (lanes D-G: 10, 50, 200, and 400 ng/ml, respectively) or 72 h (Figure 17, lane H: 400 ng/ml). Total RNA was extracted, subjected to 1.1% formaldehyde-agarose gel electrophoresis, transferred to a nylon membrane and hybridized with $^{32}P$-labeled PCR probe corresponding to $\alpha_v$. As demonstrated in Figure 17, exposure of the breast carcinoma cells for 48 h to 10 and 50 ng/ml Halofuginone resulted in up regulation of the $\alpha_v$ mRNA (Figure 17, lanes D & E). This effect was minimal at higher concentrations (200-400 ng/ml) of Halofuginone (Figure 17, lanes F-H). Next, RT-PCR was used to analyze the effect of Halofuginone on expression of the $ß_3$ and $ß_5$ integrin chains by the MDA 435 breast carcinoma cells. As shown in Figure 18, Halofuginone inhibited the expression of the $ß_3$ mRNA in a dose-dependent manner, yielding an almost complete inhibition at 200 ng/ml (Figure 18, lane 1: control; lanes 2-5: 24 h exposure to 10, 50, 200 and 400 ng/ml Halofuginone, respectively). In contrast, there was no effect on expression of the $ß_5$ mRNA. As the $\alpha_v ß_3$ integrin complex plays an important role in tumor angiogenesis, the anti-angiogenic effect of Halofuginone may be mediated in part by its inhibition of the $ß_3$ gene expression.

Example 16

Suitable Formulations for Administration of Halofuginone

Halofuginone can be administered to a subject in a number of ways, which are well known in the art. Hereinafter, the term "subject" refers to the human or lower animal to whom Halofuginone was administered. For example, administration may be done topically (including ophtalmically, vaginally, rectally, intranasally), orally, or parenterally, for example by intravenous drip or intraperitoneal, subcutaneous, or intramuscular injection.

Formulations for topical administration may include but are not limited to lotions, ointments, gels, creams, suppositories, drops, liquids, sprays and powders. Conventional pharmaceutical carriers, aqueous, powder or oily bases, thickeners and the like may be necessary or desirable.

Compositions for oral administration include powders or granules, suspensions or solutions in water or non-aqueous media, sachets, capsules or tablets. Thickeners, diluents, flavorings, dispersing aids, emulsifiers or binders may be desirable.

Formulations for parenteral administration may include but are not limited to sterile aqueous solutions which may also contain buffers, diluents and other suitable additives.

Dosing is dependent on the severity of the symptoms and on the responsiveness of the subject to Halofuginone. Persons of ordinary skill in the art can easily determine optimum dosages, dosing methodologies and repetition rates.

Example 17

Method of Treatment of Malignancies

As noted above, Halofuginone has been shown to be an effective inhibitor of tumor progression by inhibiting angiogenesis. The following example is an illustration only of a method of treating malignancies with Halofuginone, and is not intended to be limiting.

The method includes the step of administering Halofuginone, in a pharmaceutically acceptable carrier as described in Example 16 above, to a subject to be treated. Halofuginone is administered according to an effective dosing methodology, preferably until a predefined endpoint is reached, such as the absence of a particular tumor marker in a sample taken from the subject.

Examples of tumors for which such a treatment would be effective include, but are not limited to, breast cancers such as infiltrating duct carcinoma of the breast, lung cancers such as small cell lung carcinoma, bone cancers, bladder cancers such as bladder carcinoma, rhabdomyosarcoma, angiosarcoma, adenocarcinoma of the colon, prostate or pancreas, squamous cell carcinoma of the cervix, ovarian cancer, malignant fibrous histiocytoma, skin cancers such as malignant melanoma, leiomyosarcoma, astrocytoma, glioma and hepatocellular carcinoma.

Example 18

Method of Manufacture of a Medicament Containing Halofuginone

The following is an example of a method of manufacturing Halofuginone. First, Halofuginone is synthesized in accordance with good pharmaceutical manufacturing practice. Examples of methods of synthesizing Halofuginone, and related quinazolinone derivatives, are given in U.S. Patent No. 3,338,909. Next, Halofuginone is placed in a suitable pharmaceutical carrier, as described in Example 16 above, again in accordance with good pharmaceutical manufacturing practice.

While the invention has been described with respect to a limited number of embodiments, it will be appreciated that many variations, modifications and other applications of the invention may be made.

## Claims

1. Use of Halofuginone and pharmaceutically acceptable salts thereof for preparing a medicament for treating a tumor selected from the group consisting of breast cancer, lung cancer, bladder cancer, rhabdomyosarcoma, angiosarcoma, adenocarcinoma of the colon, prostate or pancreas, squamous cell carcinoma of the cervix, ovarian cancer, malignant fibrous histiocytoma, skin cancer, astrocytoma, glioma and hepatocellularcarcinoma.

2. Use of Halofuginone and pharmaceutically acceptable salts thereof for preparing a medicament for inhibiting cell proliferation enabled by a deposition of an extracellular matrix in a tumor selected from the group consisting of breast cancer, lung cancer, bladder cancer, rhabdomyosarcoma, angiosarcoma, adenocarcinoma of the colon, prostate or pancreas, squamous cell carcinoma of the cervix, ovarian cancer, malignant fibrous histiocytoma, skin cancer, astrocytoma, glioma and hepatocellularcarcinoma.

3. Use of Halofuginone and pharmaceutically acceptable salts thereof for preparing a medicament for inhibiting metastasis in a tumor selected from the group consisting of breast cancer, lung cancer, bladder cancer, rhabdomyosarcoma, angiosarcoma, adenocarcinoma of the colon, prostate or pancreas, squamous cell carcinoma of the cervix, ovarian cancer, malignant fibrous histiocytoma, skin cancer, astrocytoma, glioma and hepatocellularcarcinoma.

4. Use according to any one of the claims 1 to 3, wherein said breast cancer is infiltrating duct carcinoma of the breast.

5. Use according to any one of the claims 1 to 3, wherein said bladder cancer is bladder carcinoma.

6. Use according to any one of the claims 1 to 3, wherein said skin cancer is malignant melanoma.

## Patentansprüche

1. Verwendung von Halofuginon und pharmazeutisch annehmbaren Salzen davon für die Herstellung eines Medikaments zum Behandeln eines Tumors, der ausgewählt ist aus der Gruppe bestehend aus Brustkrebs, Lungenkrebs, Blasenkrebs, Rhabdomyosarkom, Angiosarkom, Adenokarzinom des Colons, der Prostata oder des Pankreas, Plattenepithelkarzinom der Cervix, Ovarialkrebs, malignem, fibrösem Histiozytom, Hautkrebs, Astrozytom, Gliom und hepatozellulärem Karzinom.

2. Verwendung von Halofuginon und pharmazeutisch annehmbaren Salzen davon für die Herstellung eines Medikaments zum Hemmen von Zellproliferation, die durch die Ablagerung einer extrazellulären Matrix in einem Tumor, der ausgewählt ist aus der Gruppe bestehend aus Brustkrebs, Lungenkrebs, Blasenkrebs, Rhabdomyosarkom, Angiosarkom, Adenokarzinom des Colons, der Prostata oder des Pankreas, Plattenepithelkarzinom der Cervix, Ovarialkrebs, malignem, fibrösem Histiozytom, Hautkrebs, Astrozytom, Gliom und hepatozellulärem Karzinom, ermöglicht wird.

3. Verwendung von Halofuginon und pharmazeutisch annehmbaren Salzen davon für die Herstellung eines Medikaments zum Hemmen von Metastasierung bei einem Tumor, der ausgewählt ist aus der Gruppe bestehend aus Brustkrebs, Lungenkrebs, Blasenkrebs, Rhabdomyosarkom, Angiosarkom, Adenokarzinom des Colons, der Prostata oder des Pankreas, Plattenepithelkarzinom der Cervix, Ovarialkrebs, malignem, fibrösem Histiozytom, Hautkrebs, Astrozytom, Gliom und hepatozellulärem Karzinom.

4. Verwendung gemäß einem der Ansprüche 1 bis 3, wobei es sich bei besagtem Brustkrebs um ein infiltrierendes duktales Karzinom der Mamma handelt.

5. Verwendung gemäß einem der Ansprüche 1 bis 3, wobei es sich bei besagtem Blasenkrebs um ein Blasenkarzinom handelt.

6. Verwendung gemäß einem der Ansprüche 1 bis 3, wobei es sich bei besagtem Hautkrebs um ein malignes Melanom handelt.

## Revendications

1. Utilisation d'halofuginone et des sels pharmaceutiquement acceptables de celle-ci pour préparer un médicament destiné au traitement d'une tumeur choisie dans le groupe consistant en le cancer du sein, le cancer du poumon, le cancer de la vessie, le rhabdomyosarcome, l'angiosarcome, l'adénocarcinome du côlon, de la prostate ou du pancréas, l'épithélioma spinocellulaire, du col de l'utérus, le cancer des ovaires, l'histiocytome fibreux malin, le cancer de la peau, l'astrocytome, le gliome et le cancer primitif du foie.

2. Utilisation d'halofuginone et des sels pharmaceutiquement acceptables de celle-ci pour préparer un médicament destiné à l'inhibition de la prolifération de cellules activée par un dépôt d'une matrice extracellulaire dans une tumeur choisie dans le groupe consistant en le cancer du sein, le cancer du poumon, le cancer de la vessie, le rhabdomyosarcome, l'angiosarcome, l'adénocarcinome du côlon, de la prostate ou du pancréas, l'épithélioma spinocellulaire, du col de l'utérus, le cancer des ovaires, l'histiocytome fibreux malin, le cancer de la peau, l'astrocytome, le gliome et le cancer primitif du foie.

3. Utilisation d'halofuginone et des sels pharmaceutiquement acceptables de celle-ci pour préparer un médicament destiné à l'inhibition de la métastase dans une tumeur choisie dans le groupe consistant en le cancer du sein, le cancer du poumon, le cancer de la vessie, le rhabdomyosarcome, l'angiosarcome, l'adénocarcinome du côlon, de la prostate ou du pancréas, l'épithélioma spinocellulaire, du col de l'utérus, le cancer des ovaires, l'histiocytome fibreux malin, le cancer de la peau, l'astrocytome, le gliome et le cancer primitif du foie.

4. Utilisation selon l'une quelconque des revendications 1 à 3, dans laquelle ledit cancer du sein est le carcinome canalaire infiltrant du sein.

5. Utilisation selon l'une quelconque des revendications 1 à 3, dans laquelle ledit cancer de la vessie est le carcinome de la vessie.

6. Utilisation selon l'une quelconque des revendications 1 à 3, dans laquelle ledit cancer de la peau est un mélanome malin.

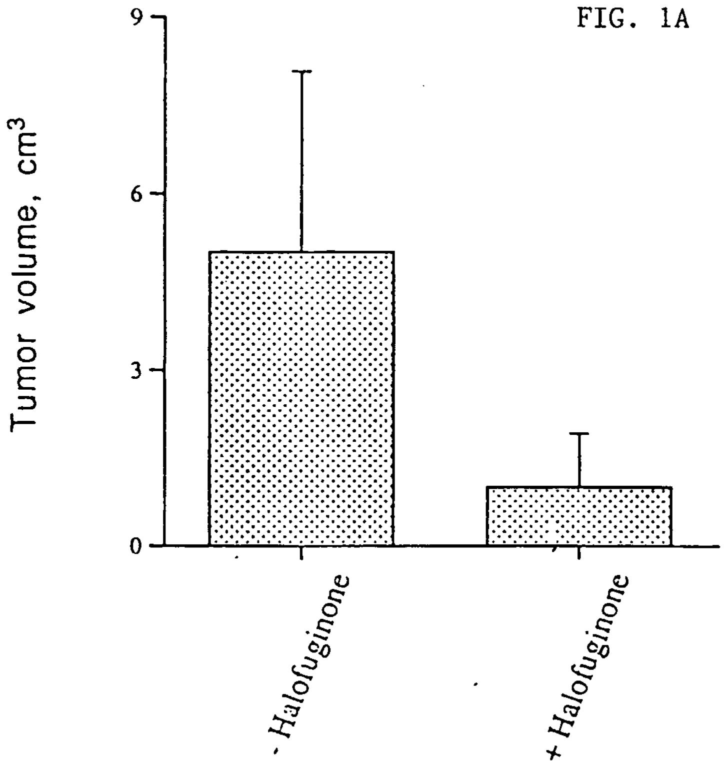
9
6
3
0
Tumor volume, cm3
- Halofuginone
+ Halofuginone

FIG. 1A

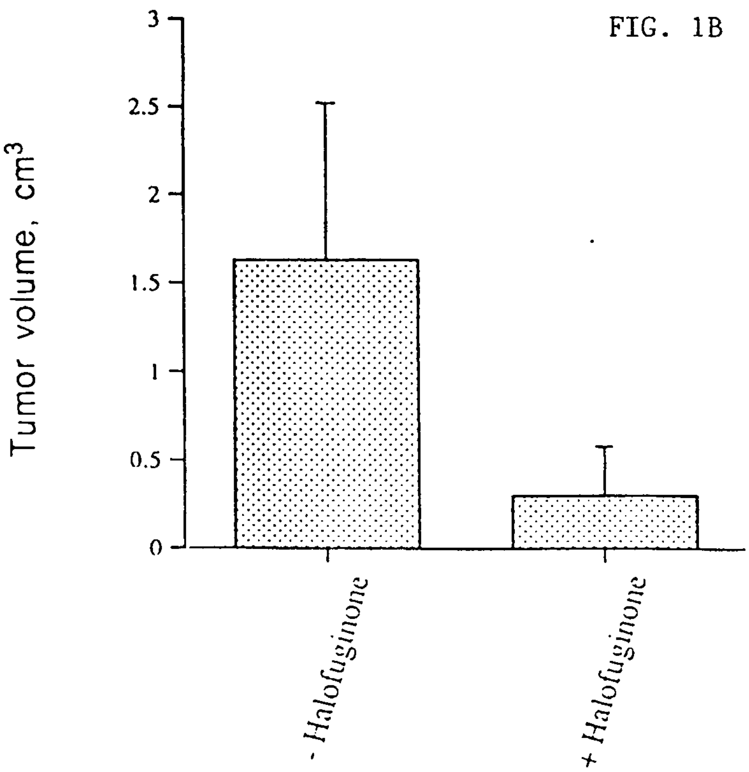
3
2.5
2
1.5
1
0.5
0
Tumor volume, cm3
- Halofuginone
+ Halofuginone

FIG. 1B

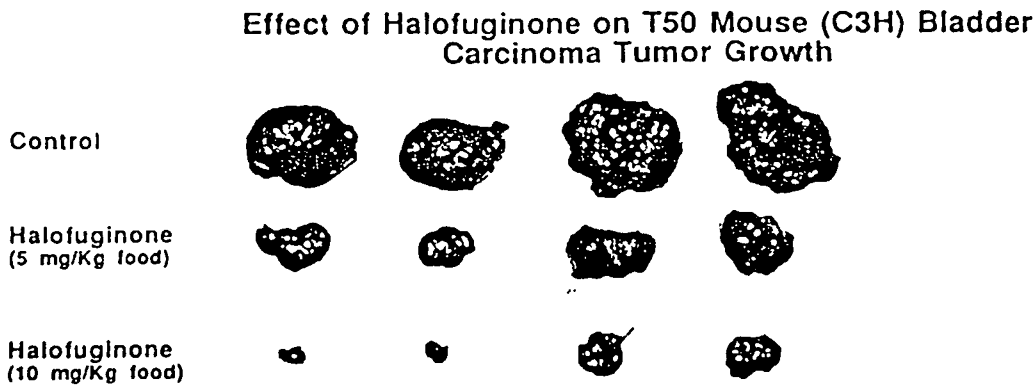
Effect of Halofuginone on T50 Mouse (C3H) Bladder Carcinoma Tumor Growth
Control
Halofuginone
(5 mg/Kg food)
Halofuginone
(10 mg/Kg food)

FIG 1 C

FIG 1 D

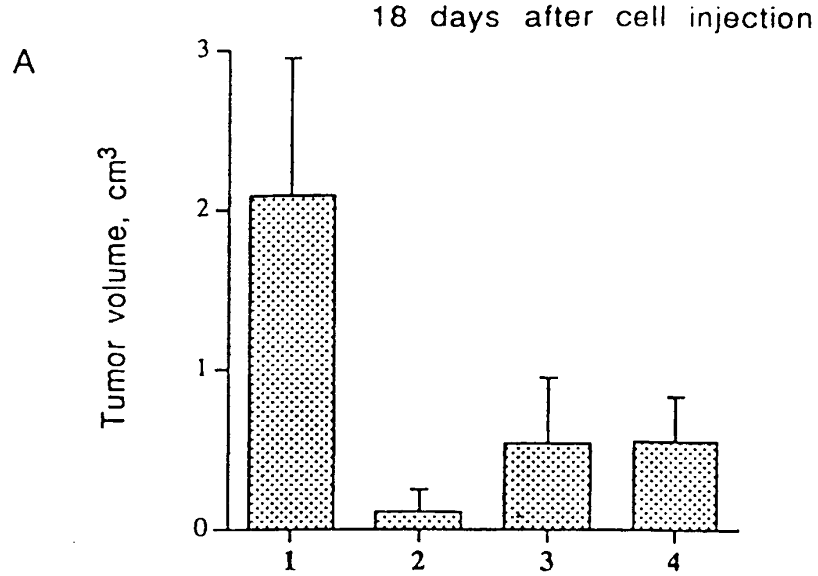


FIG 1 E

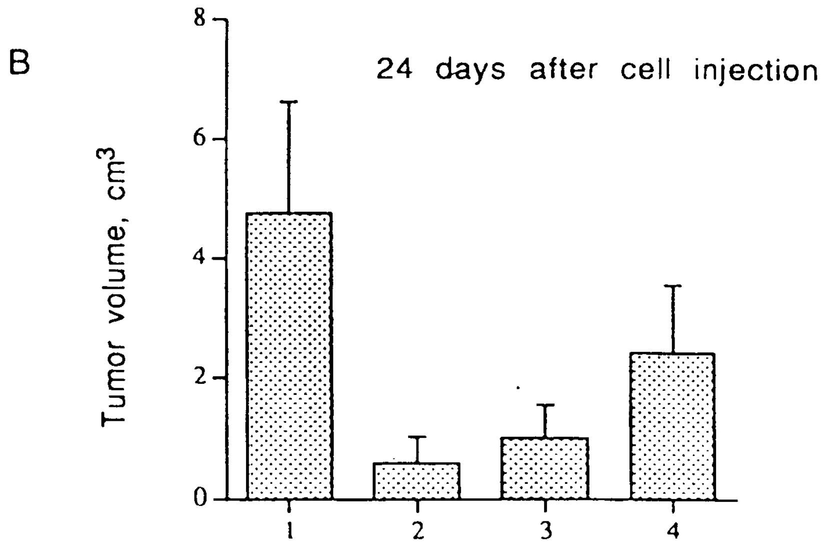


1. Control
2. Halofuginone in food administrated 2 days before cell injection
3. Halofuginone in food administrated after tumor appearance
4. Halofuginone i.p. every day starting on the day of cell injection

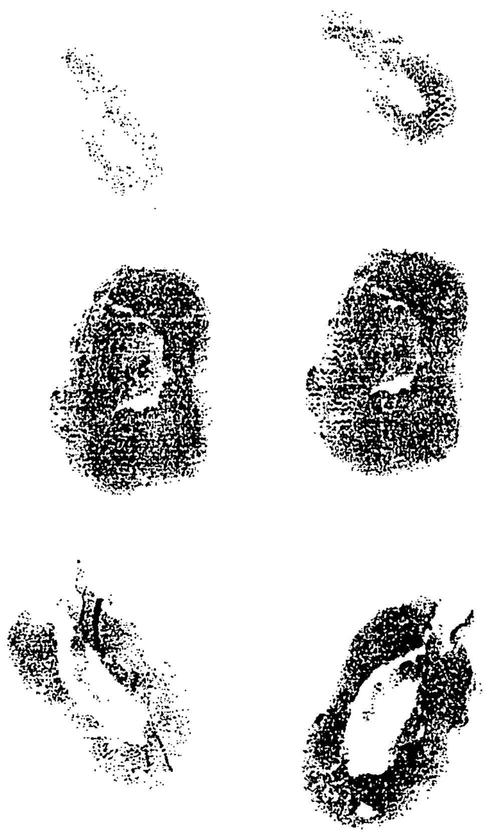

FIG 1F a

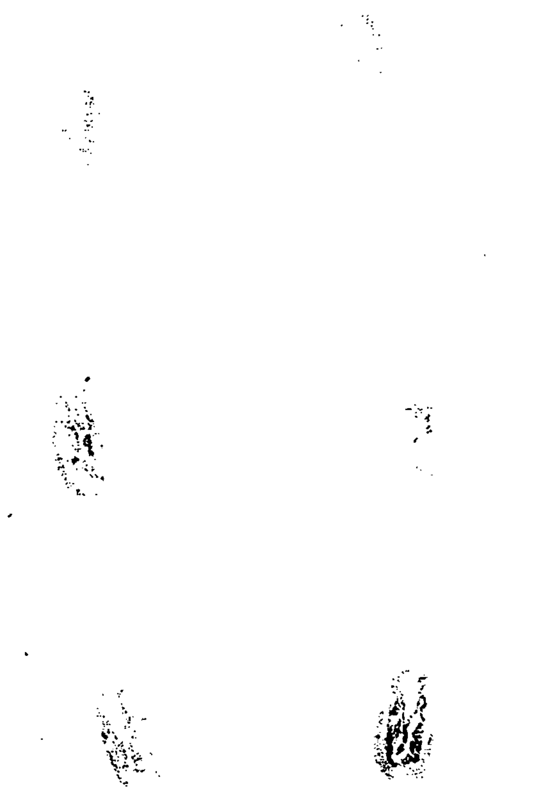

FIG 1F b

FIG 1G A1

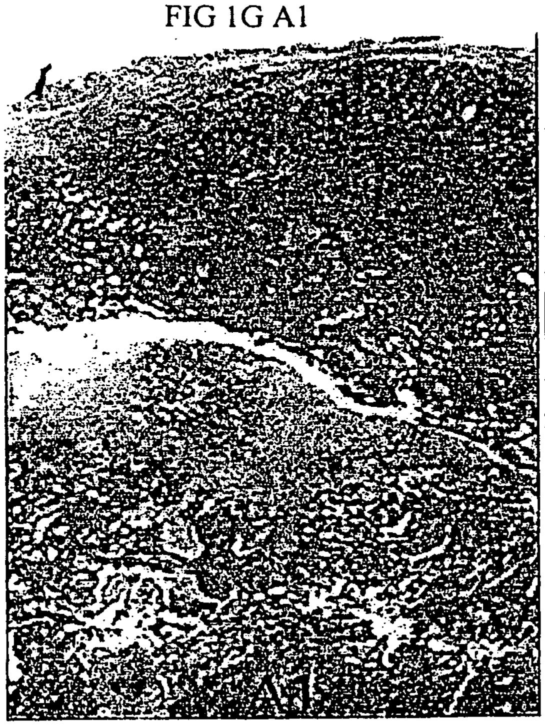


FIG 1G B1

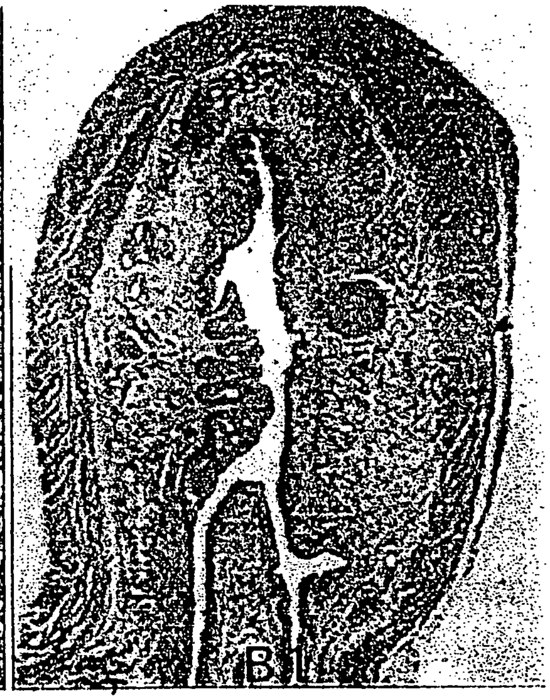


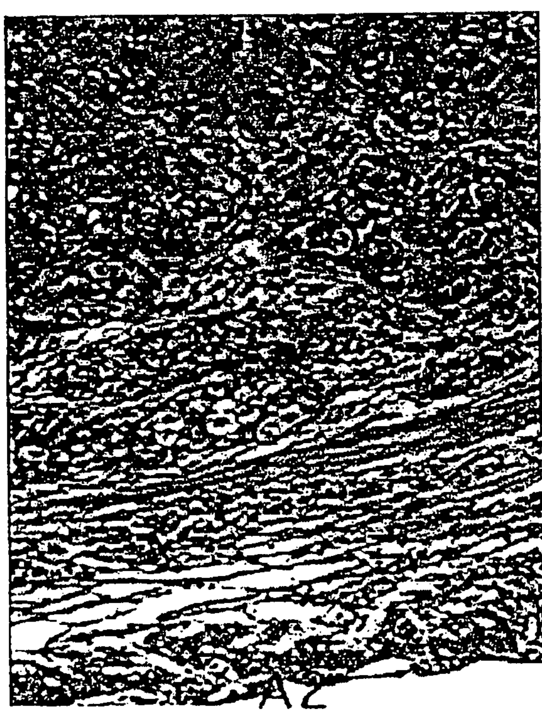


FIG 1G A2

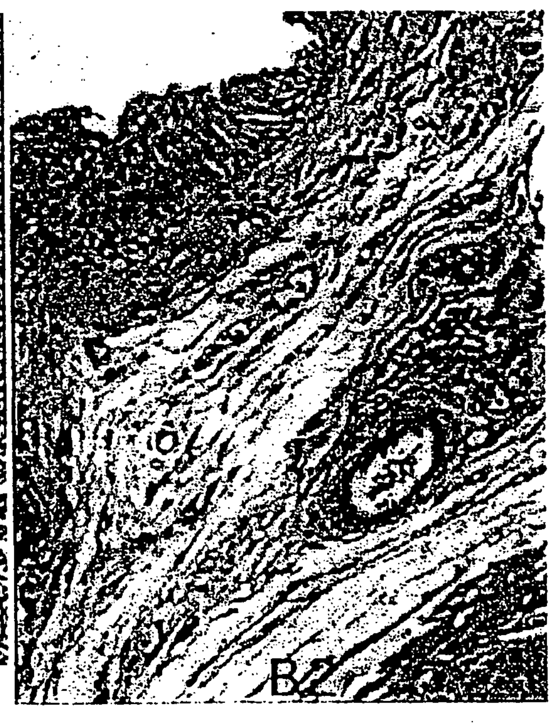


FIG 1G B2

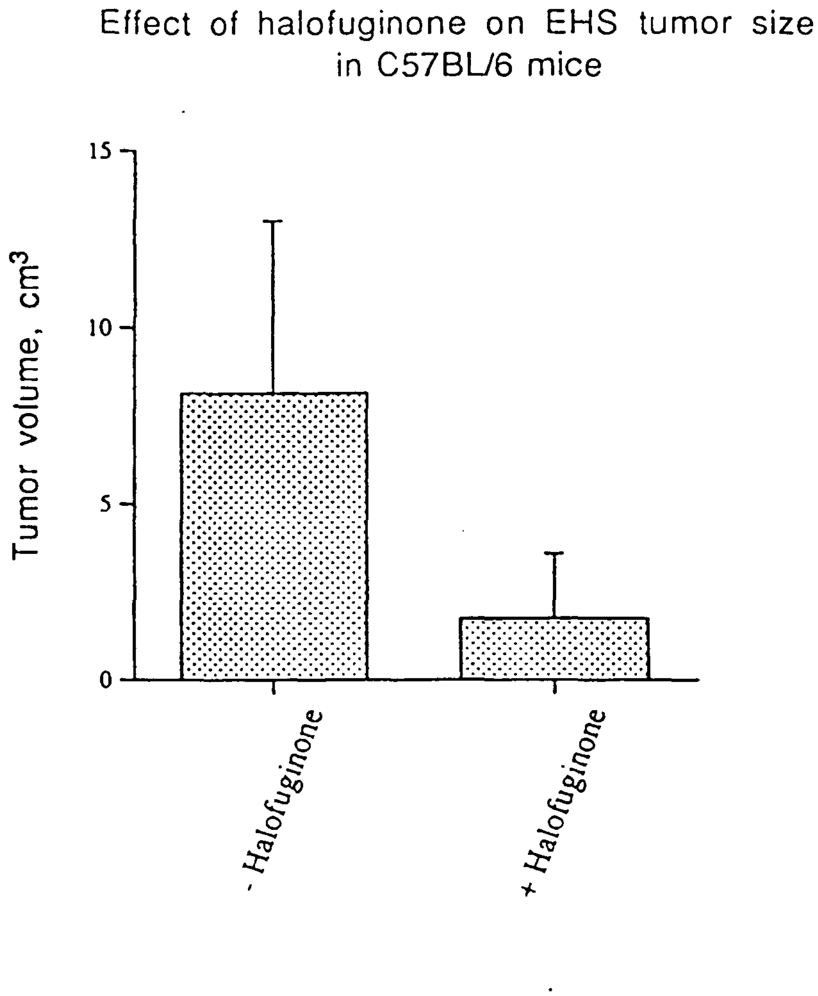
Effect of halofuginone on EHS tumor size
in C57BL/6 mice
Tumor volume, cm3
15
10
5
0
- Halofuginone
+ Halofuginone

FIG 1 H

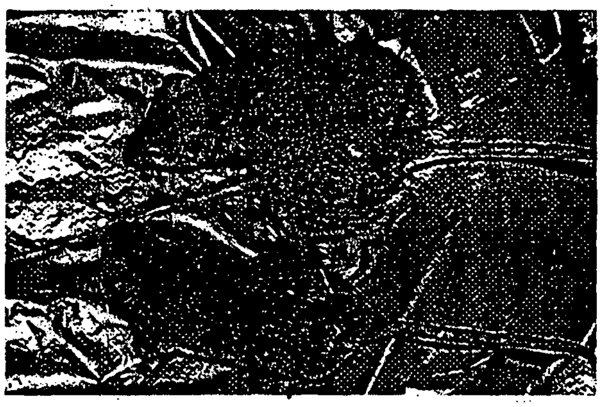

FIG 1 I

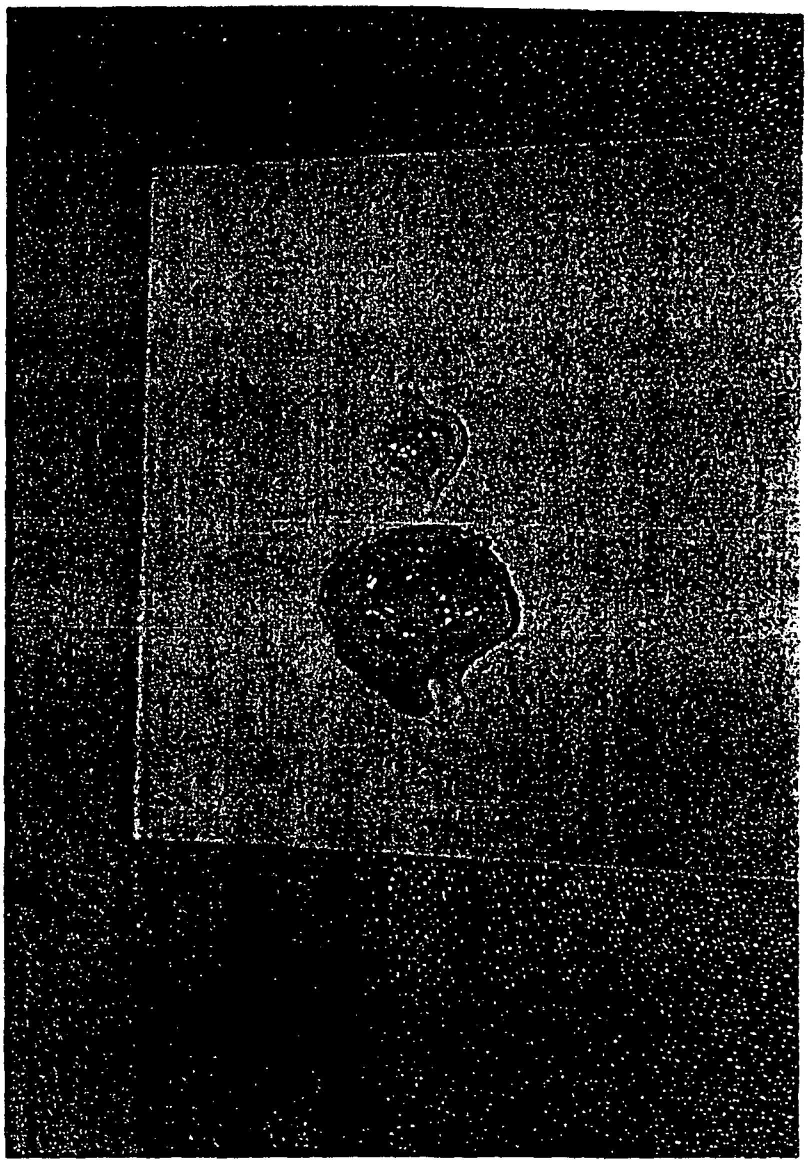

FIG 1 J

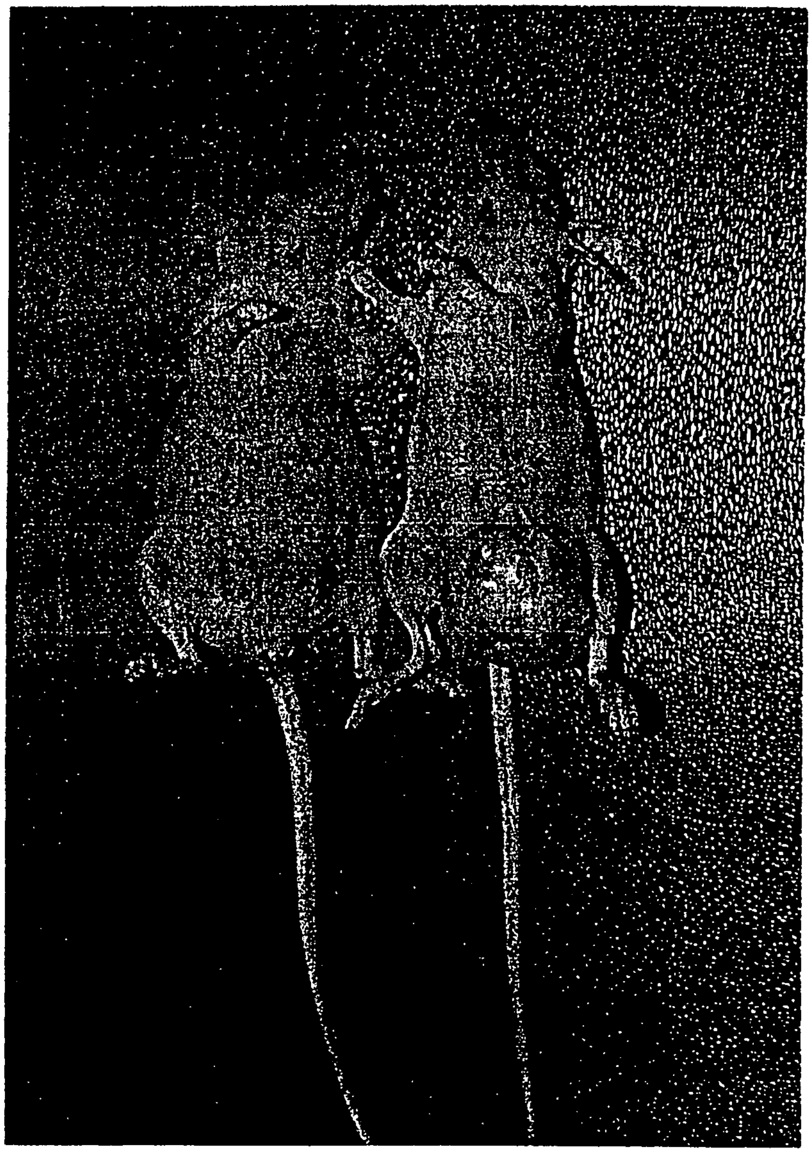

FIG 1 K

MELANOMA

+H

-H

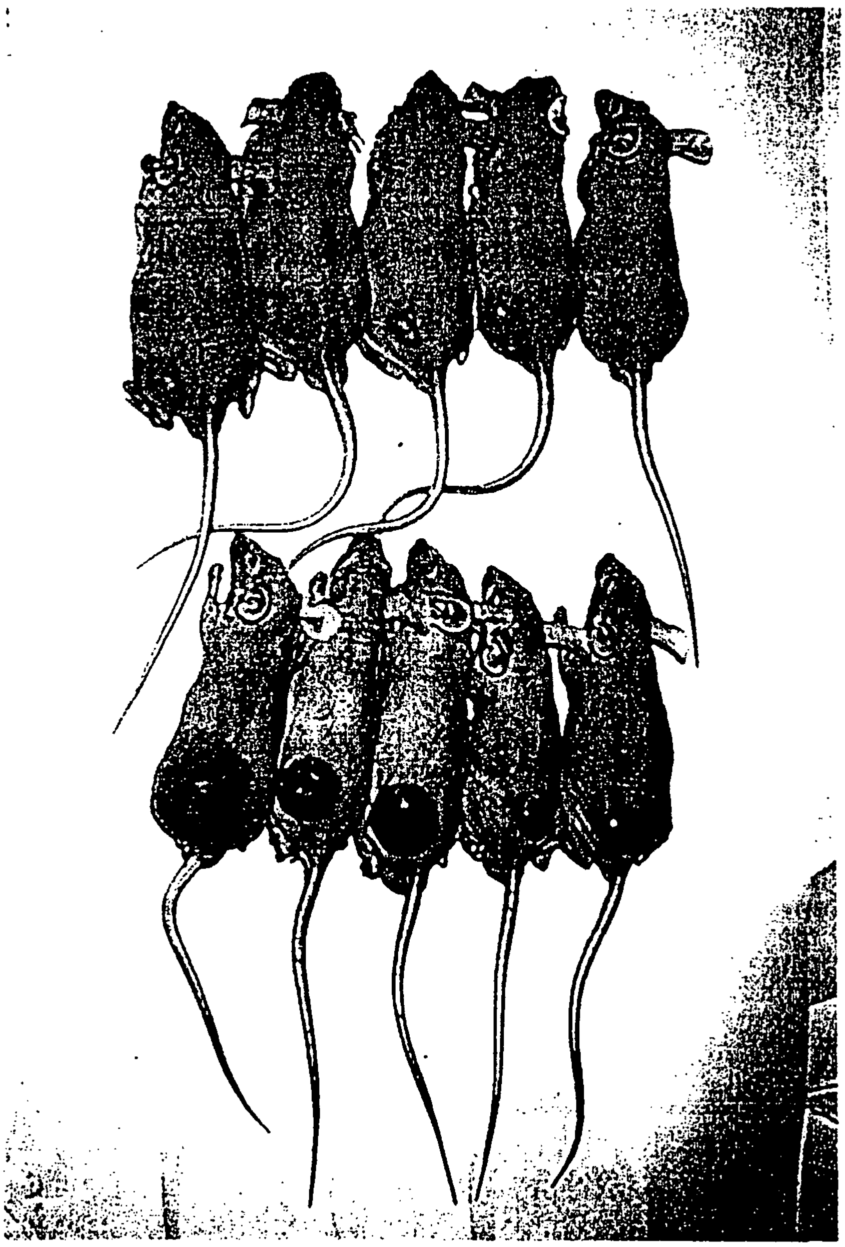

FIG 1L

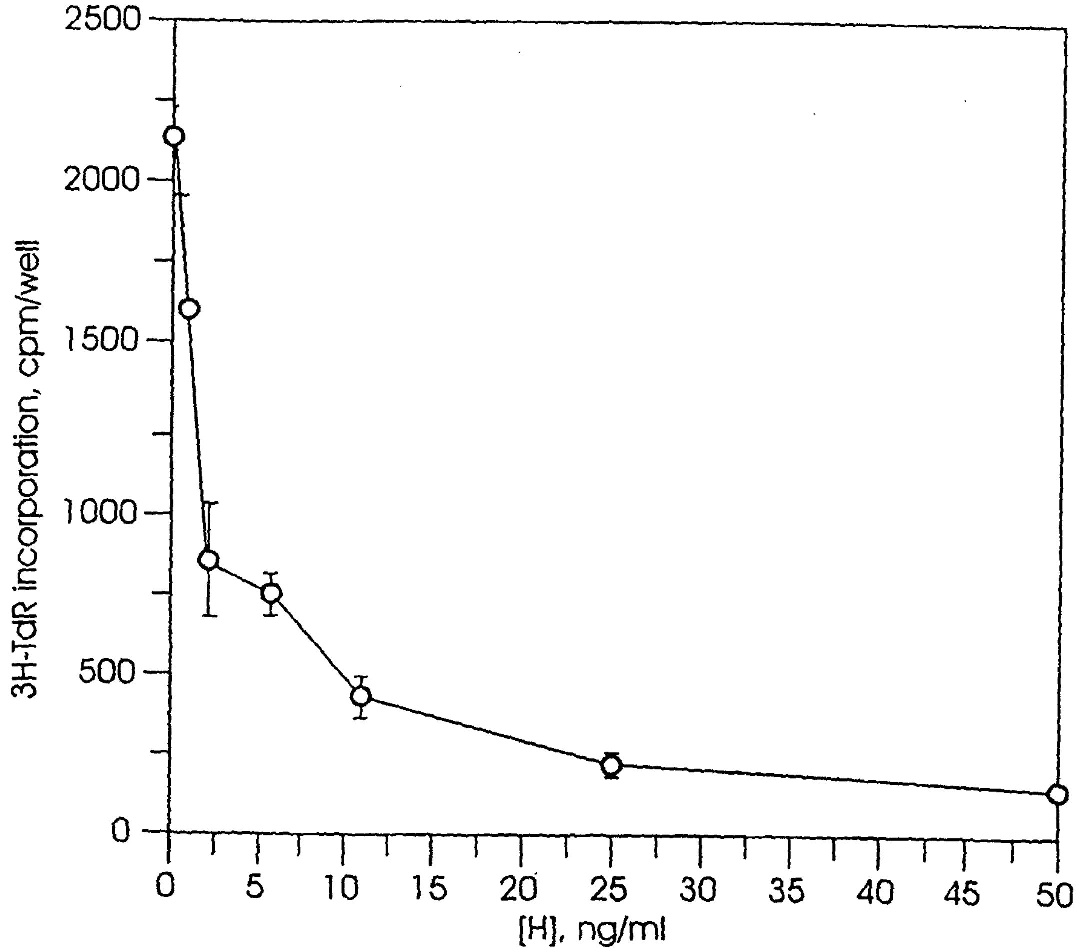
2500
2000
1500
1000
500
0
3H-TdR incorporation, cpm/well
0
5
10
15
20
25
30
35
40
45
50
[H], ng/ml

Fig. 2a

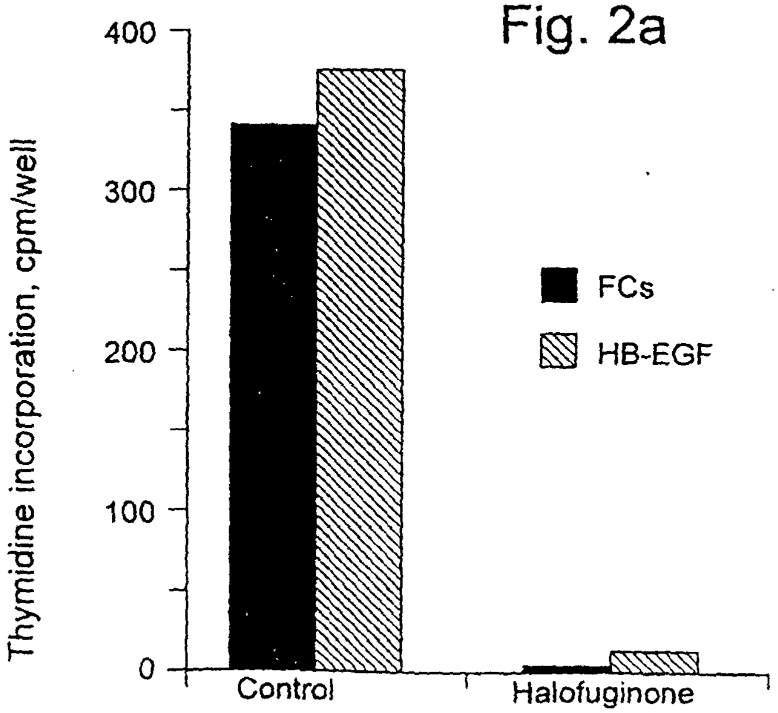
400
300
200
100
0
Thymidine incorporation, cpm/well
FCs
HB-EGF
Control
Halofuginone

Fig. 2b

Inhibition of collagenase (MMP2) activity in T50 bladder carcinoma cells by halofuginone.

Halofuginone conc. (ng/ml)
in T50 cells growth medium

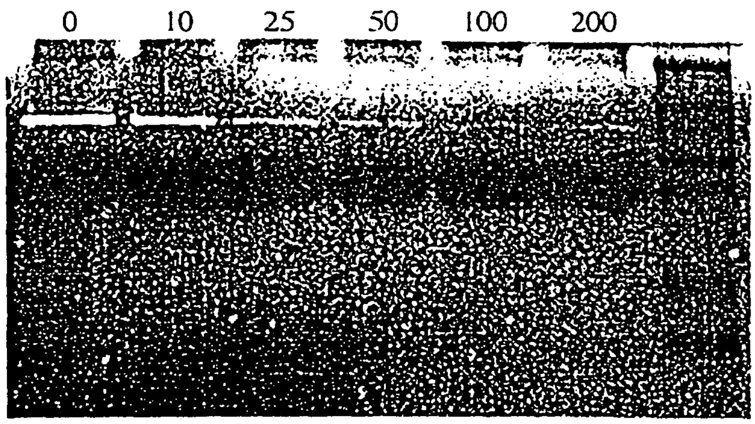


FIG 3

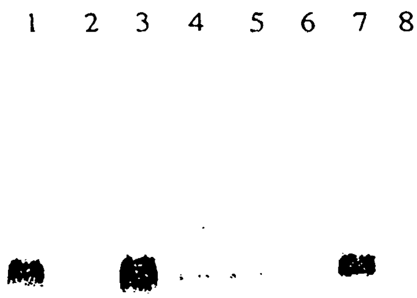


FIG 4 A

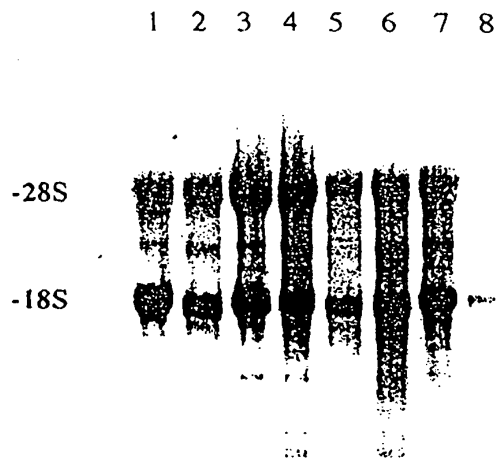


FIG 4 B

A - nothern blot hybridisation with DIG-labled H19 probe (CDP-Star detection, exp. time-2min.).
B - RNA loading on the blot, methylen blue staining befor hybridisation.

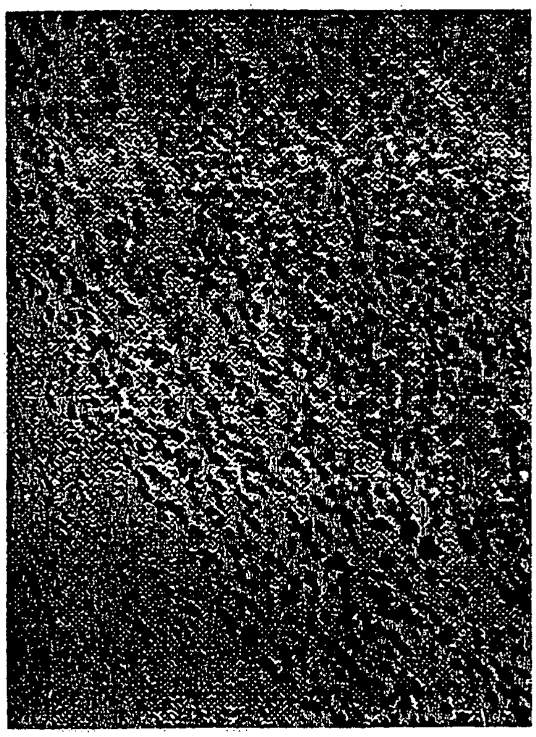

FIG 5 A

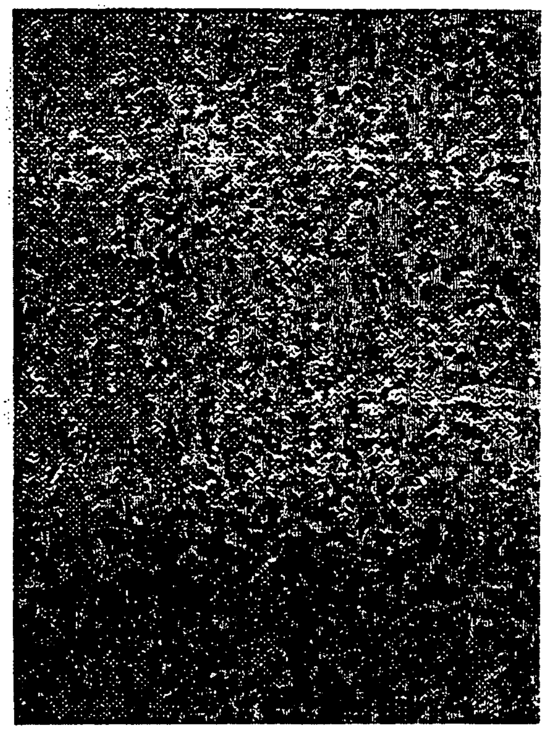

FIG 5 B

FIG 6B

FIG 6 D

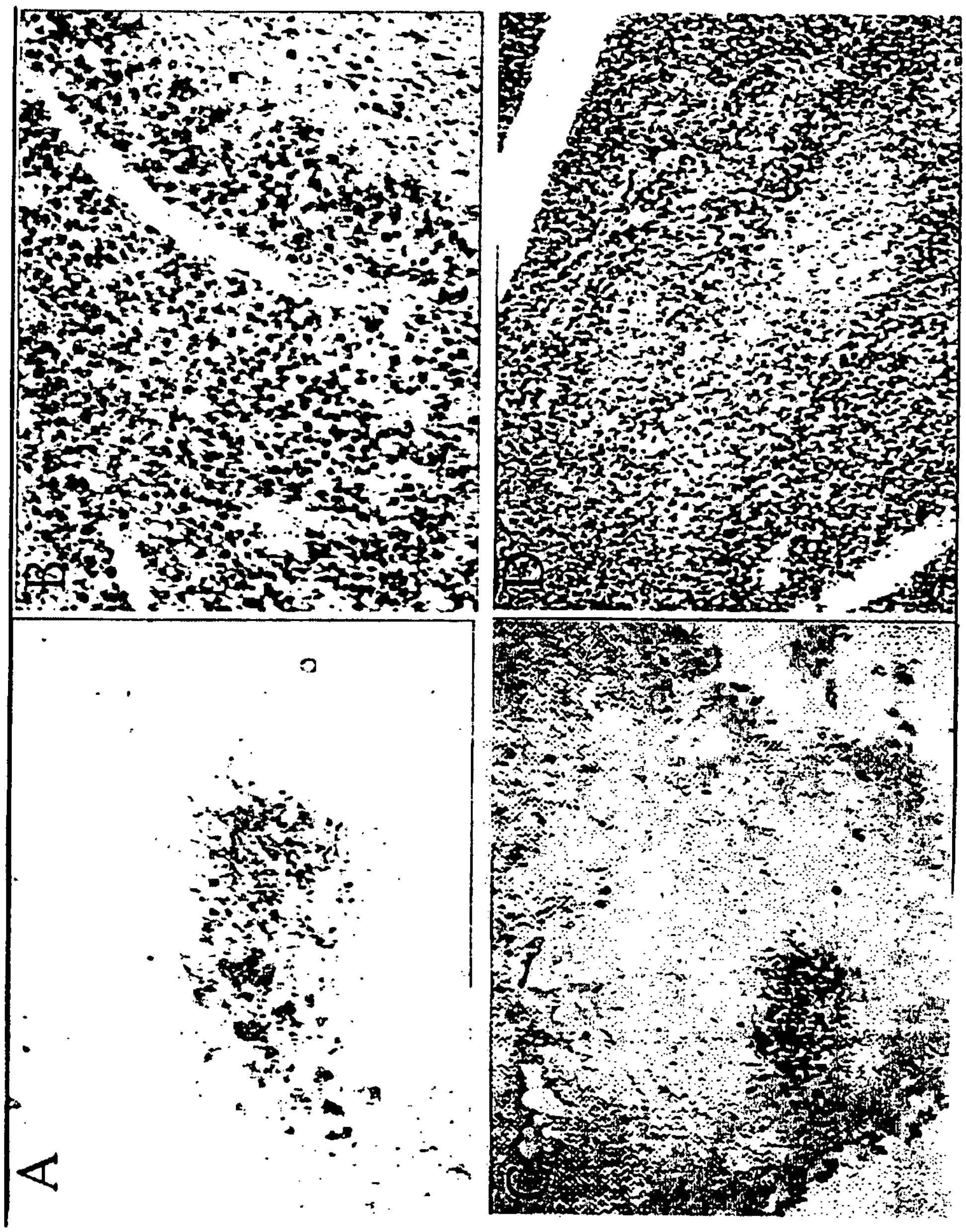

FIG 6 A

FIG 6C

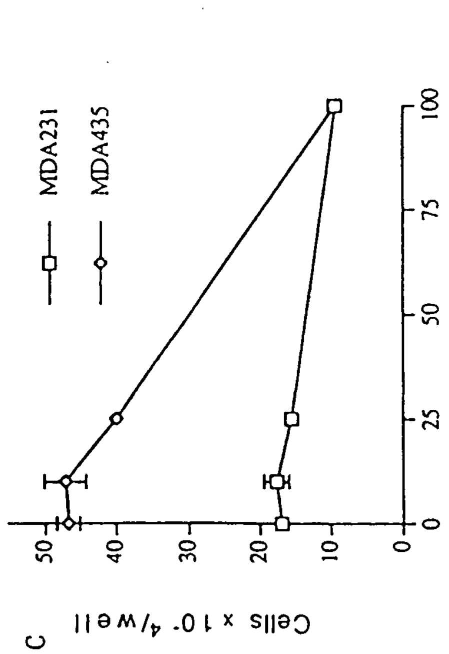


FIG 7 C

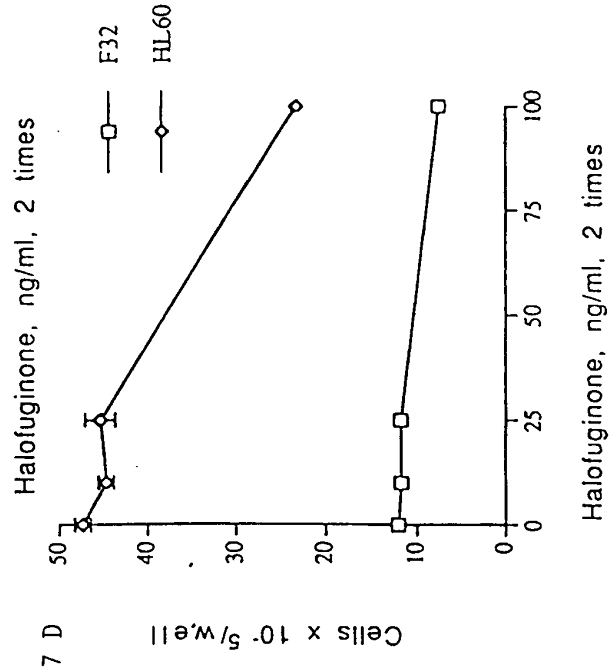


FIG 7 D

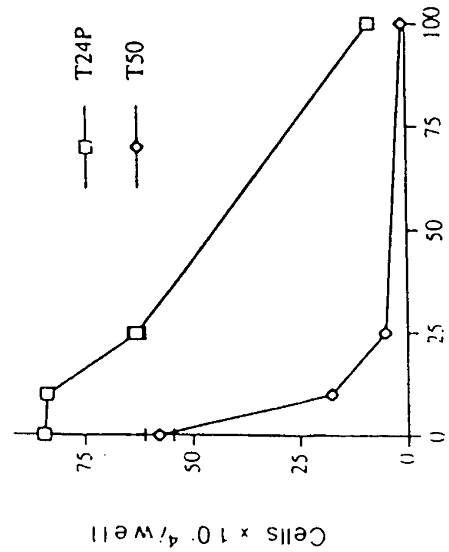


FIG 7 A

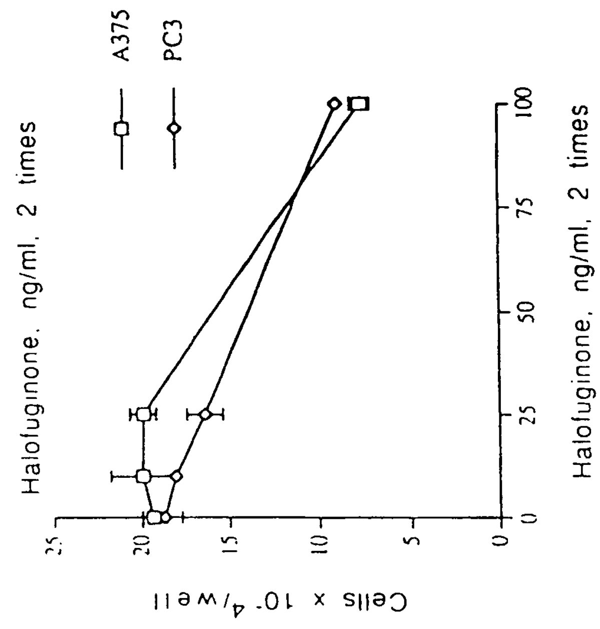


FIG 7B

Antiproliferative effect of halofuginone on vascular endothelial cells
a: cells were maintained in the absence of bFGF

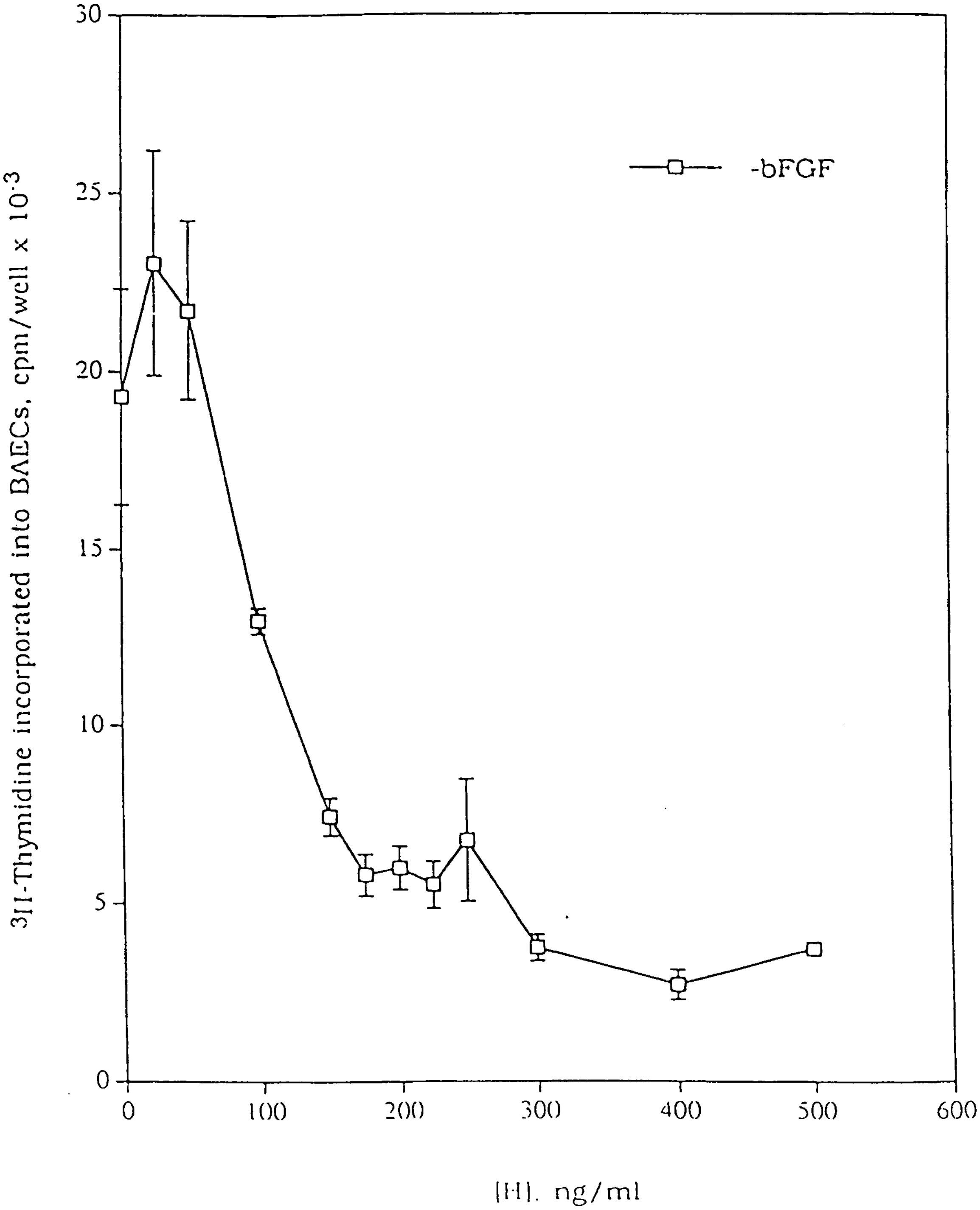


FIG 8 A

Antiproliferative effect of halofuginone on vascular endothelial cells
b: cells were maintained in the presence of bFGF

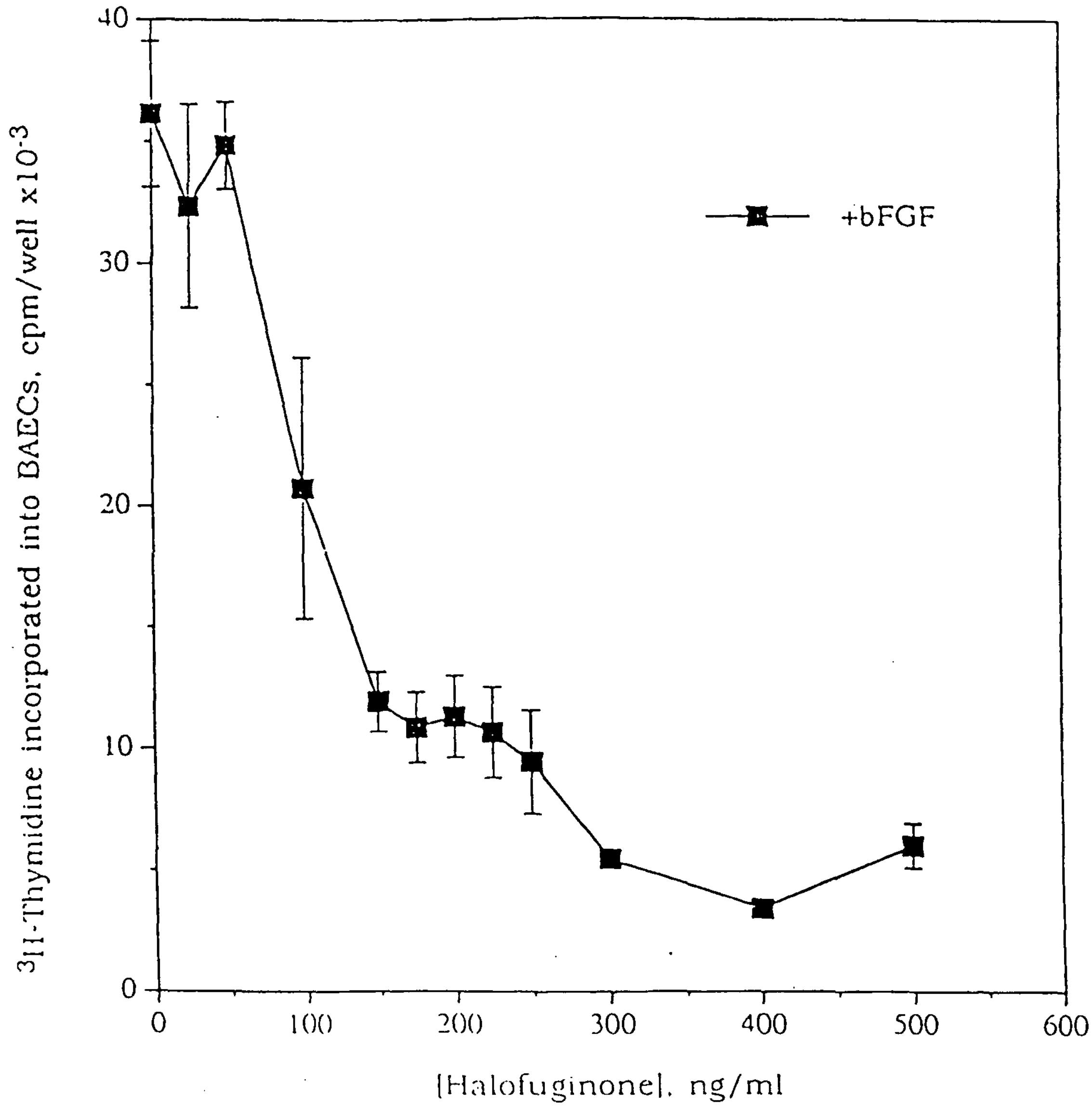


FIG 8 B

FIG 9 B

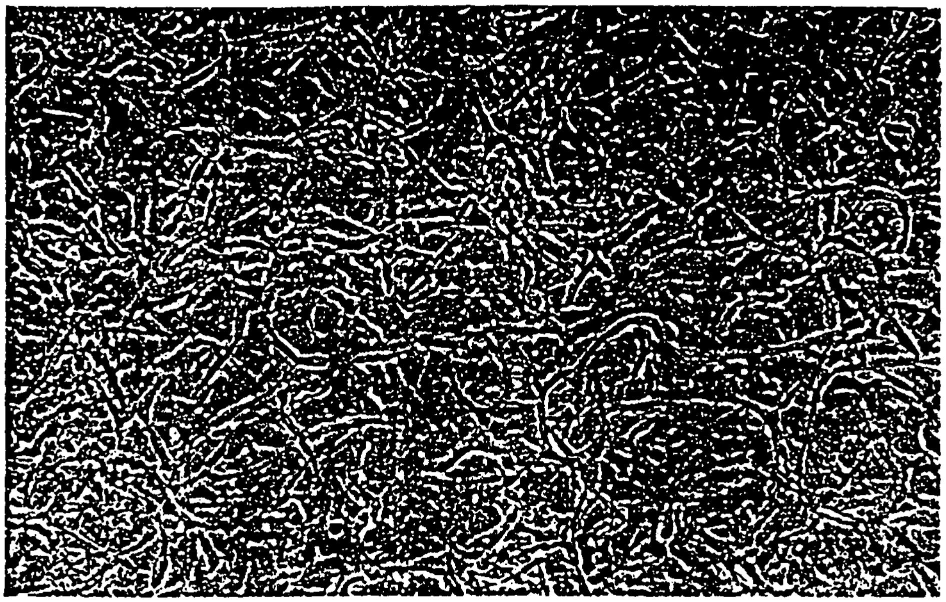

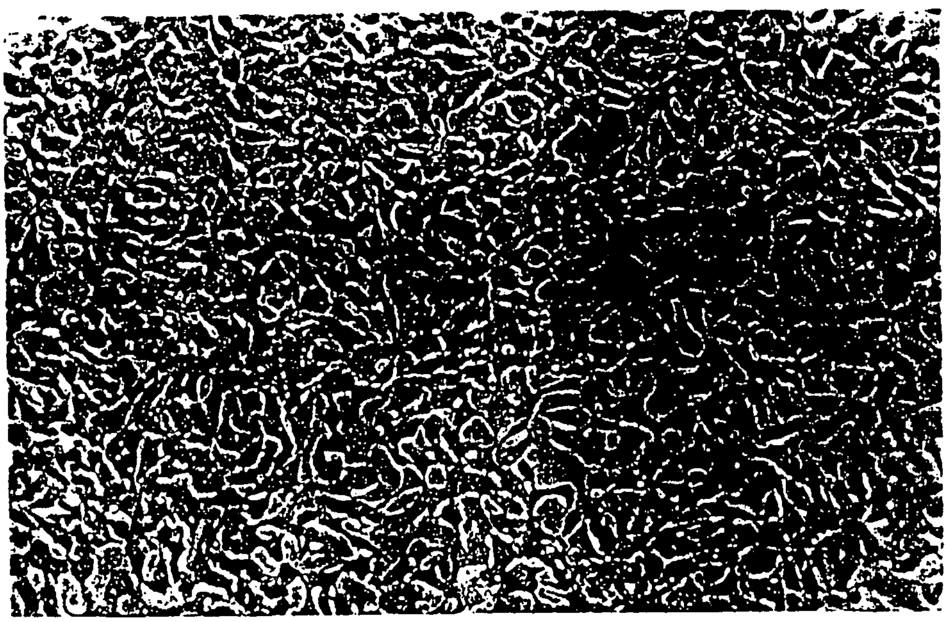

FIG 9 A

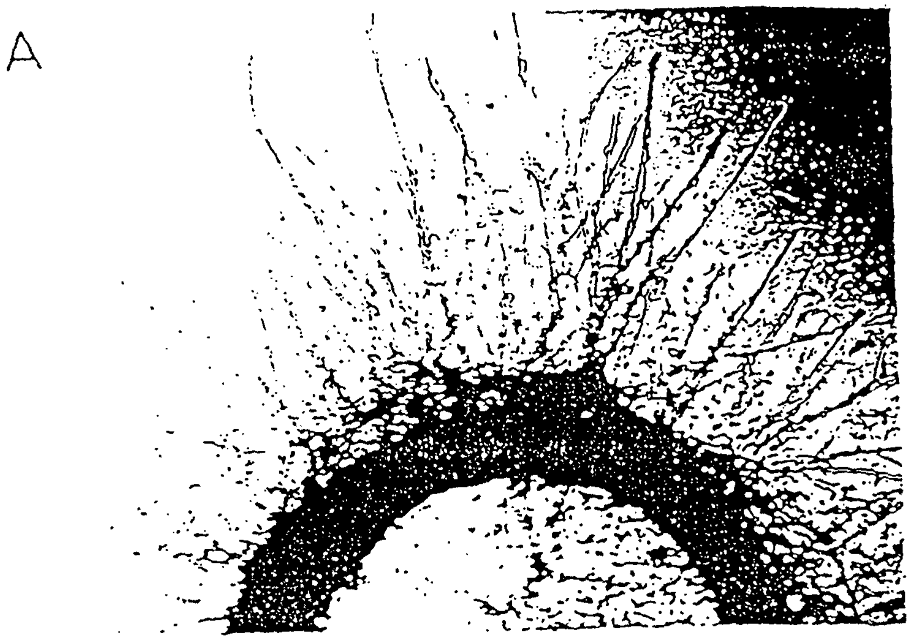

FIG 10 A

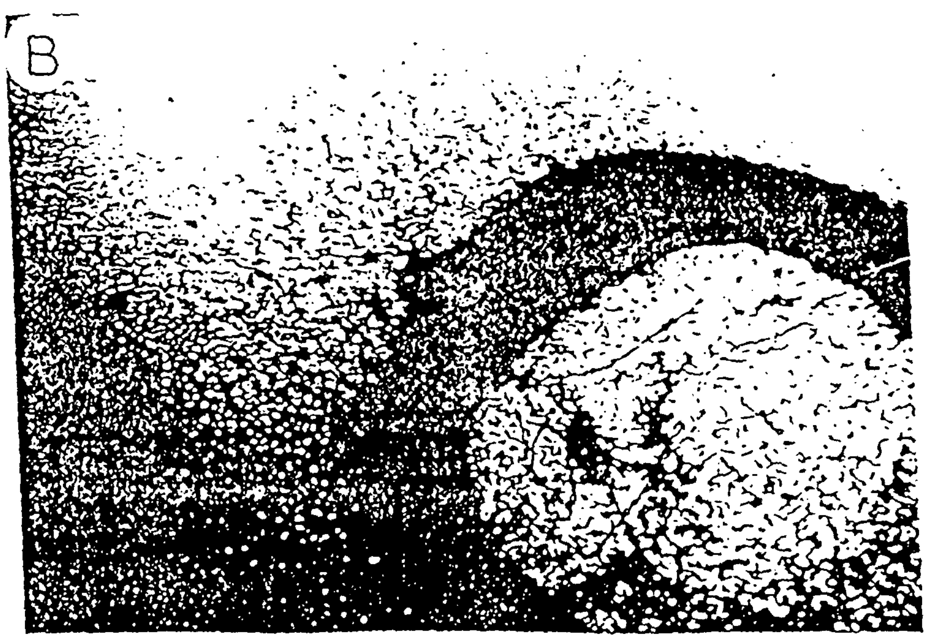

FIG 10 B

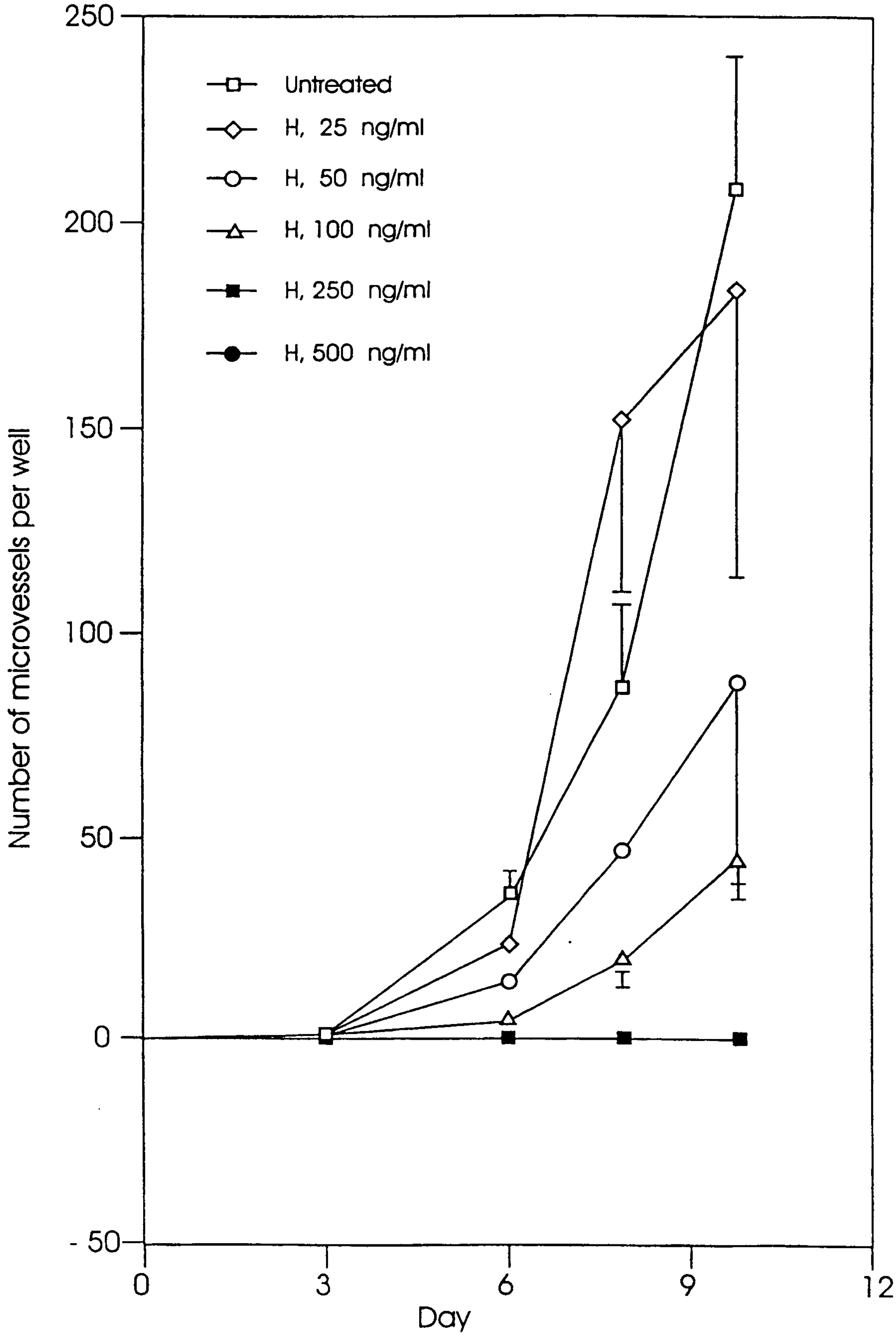
Untreated
H, 25 ng/ml
H, 50 ng/ml
H, 100 ng/ml
H, 250 ng/ml
H, 500 ng/ml
Number of microvessels per well
250
200
150
100
50
0
- 50
0
3
6
9
12
Day

Fig. 11

Reversion of the inhibitory effect of halofuginone on microvessel formation

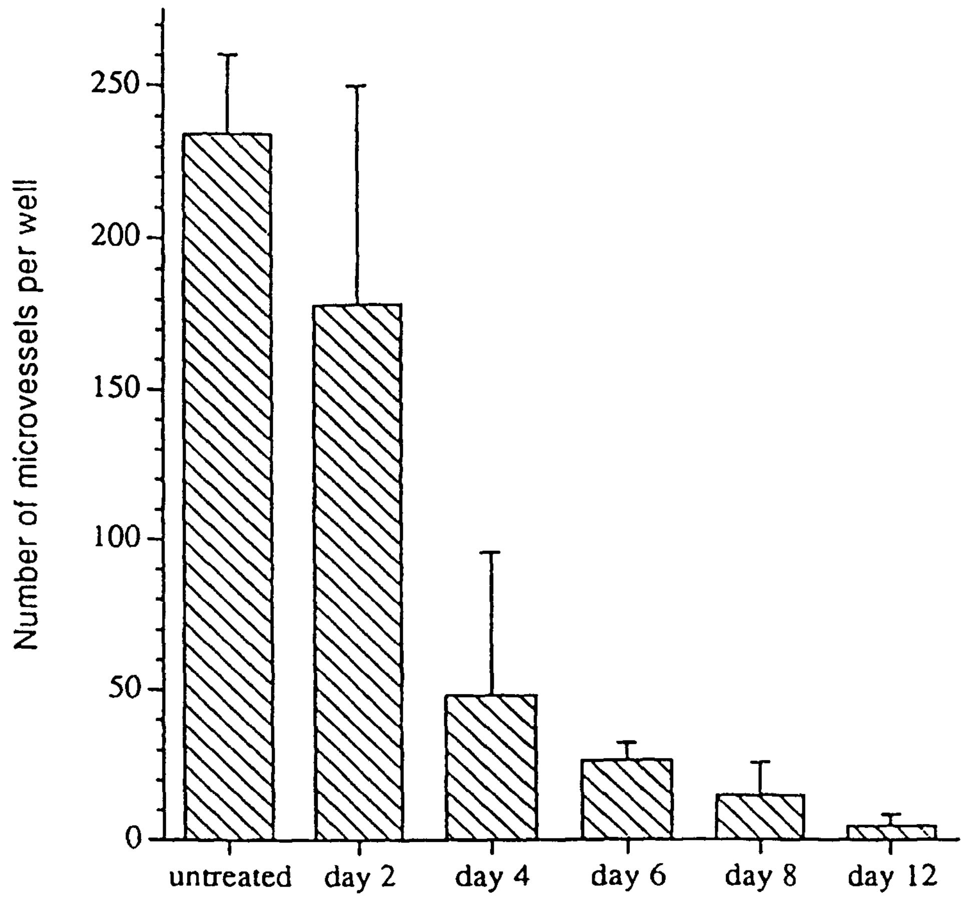


FIG 12

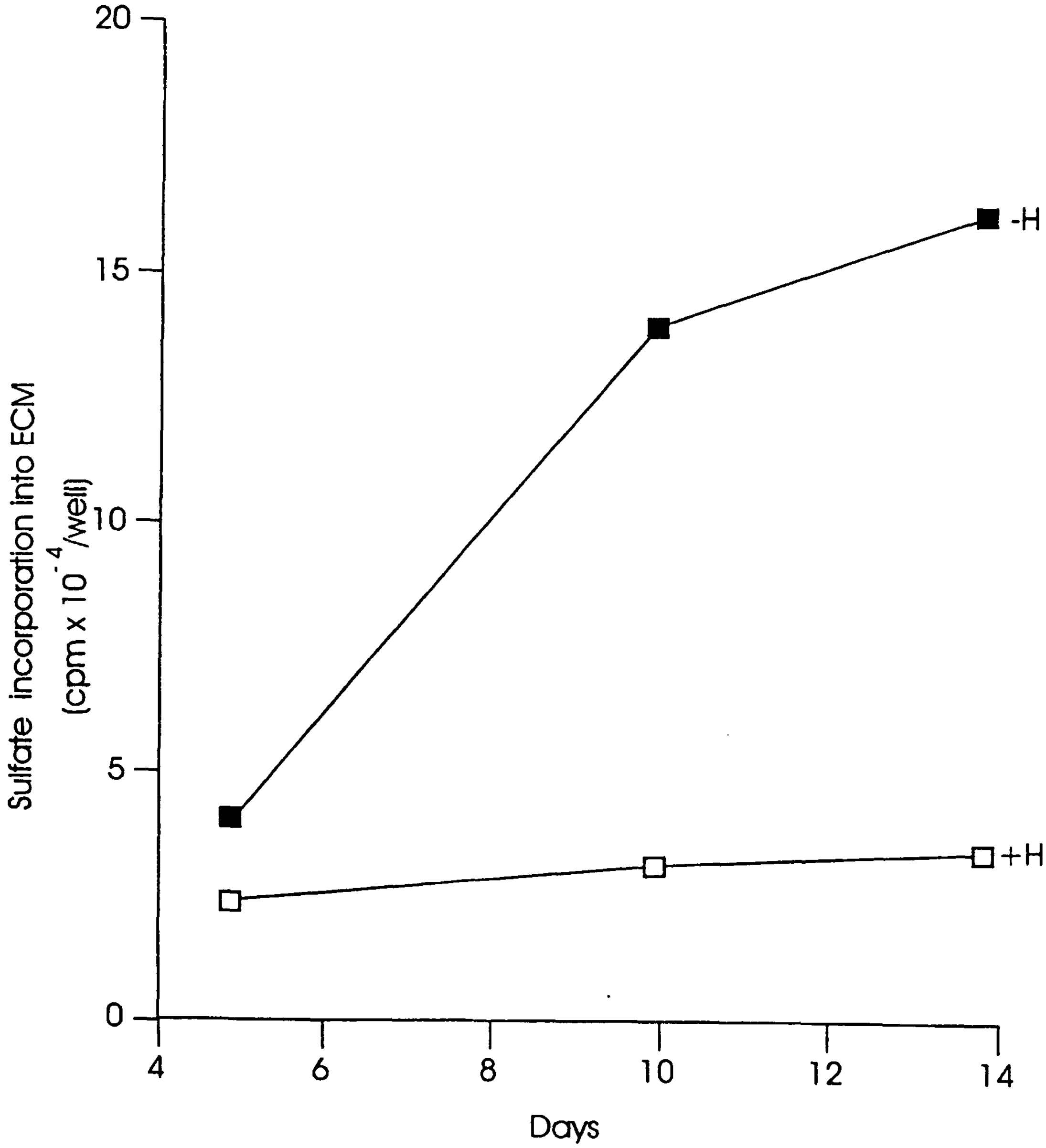
20
15
10
5
0
Sulfate incorporation into ECM
(cpm x 10^-4/well)
4
6
8
10
12
14
Days
-H
+H

Fig. 13

Effect of halofuginone on ECM production
by corneal endothelial cells

FIG 14 A

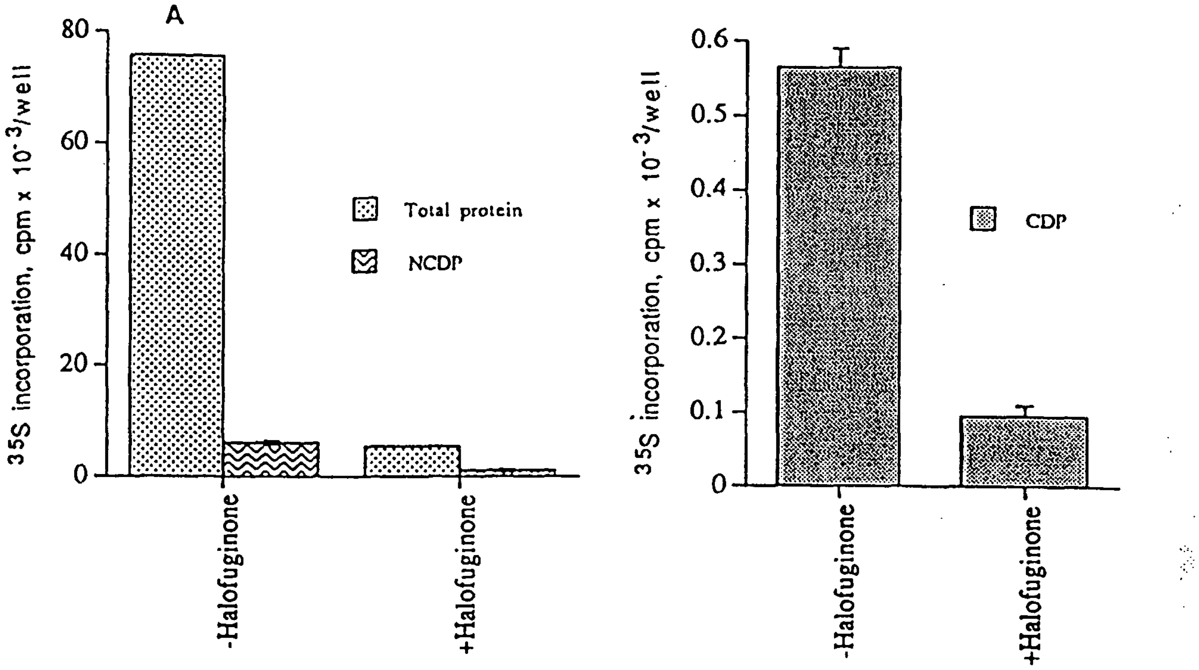


FIG 14 B

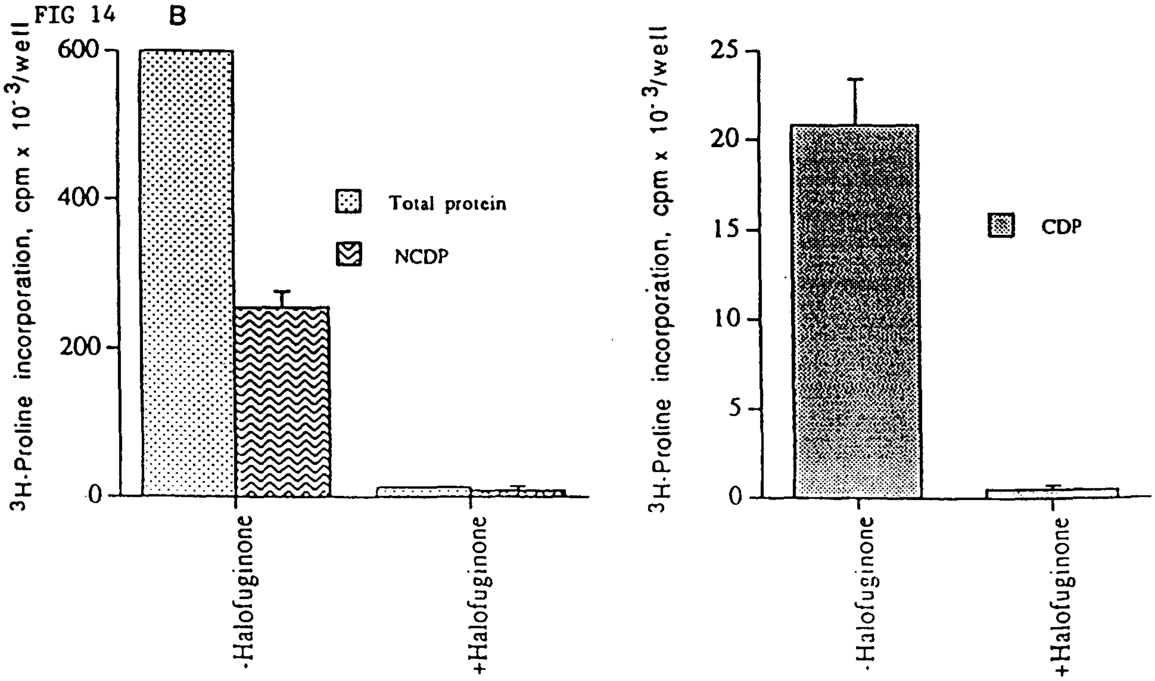

Effect of halofuginone on ECM production
by corneal endothelial cells

FIG 14 C

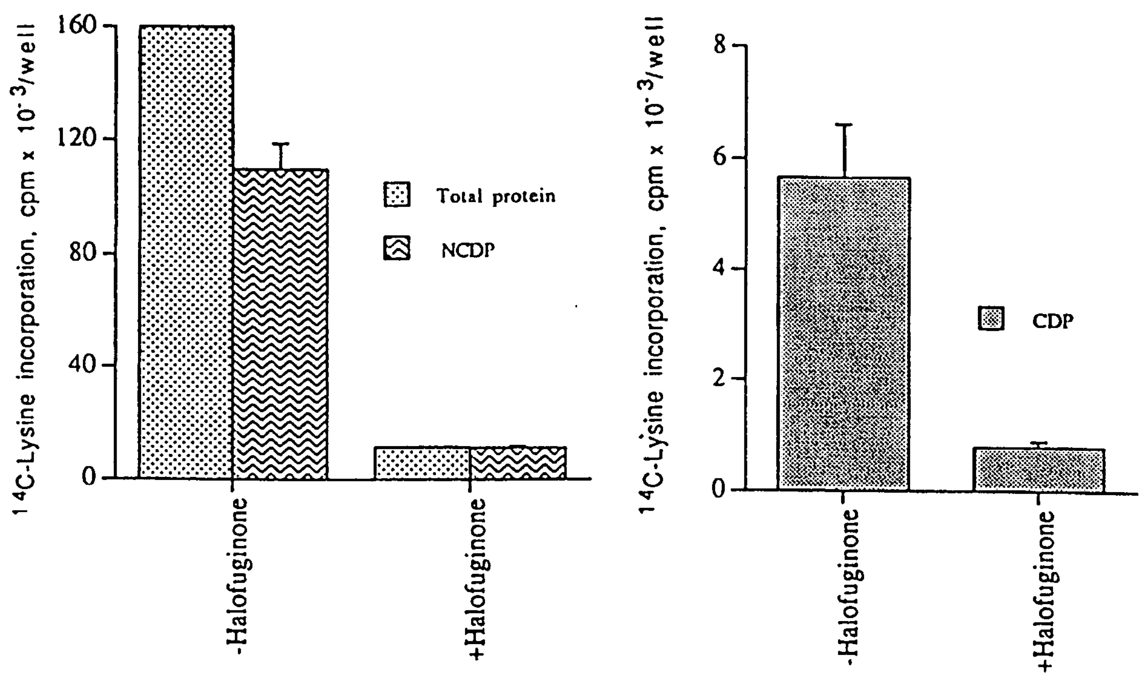


FIG 14 D

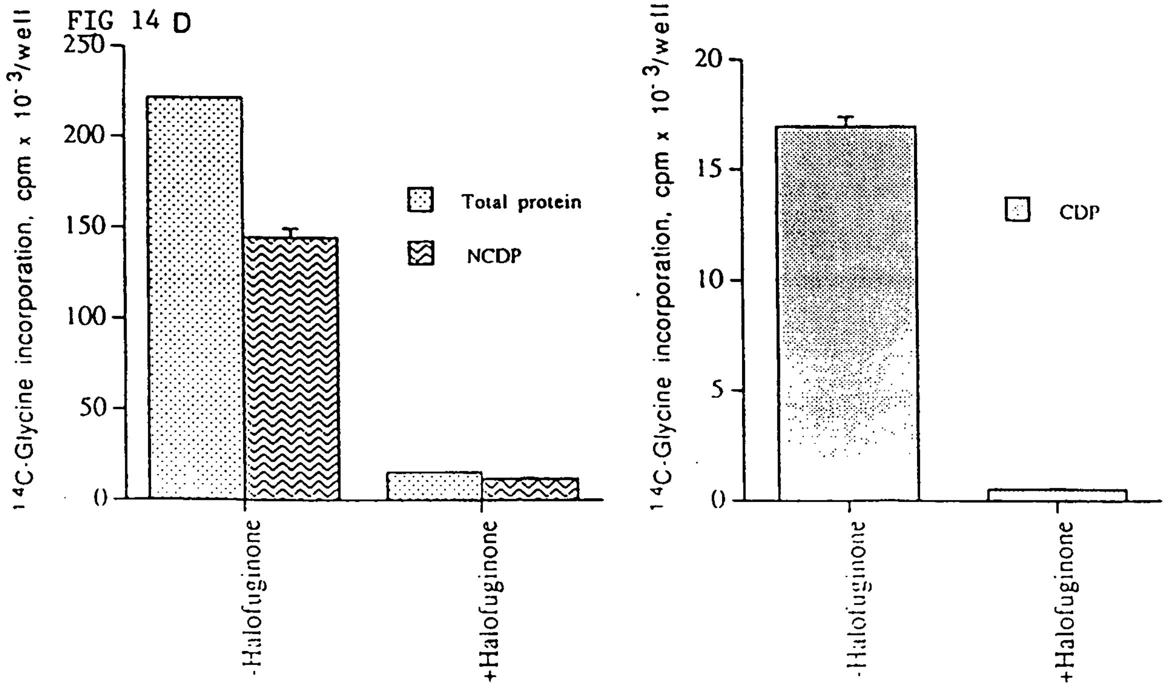

Effect of halofuginone on ECM production by rat mesangial cells

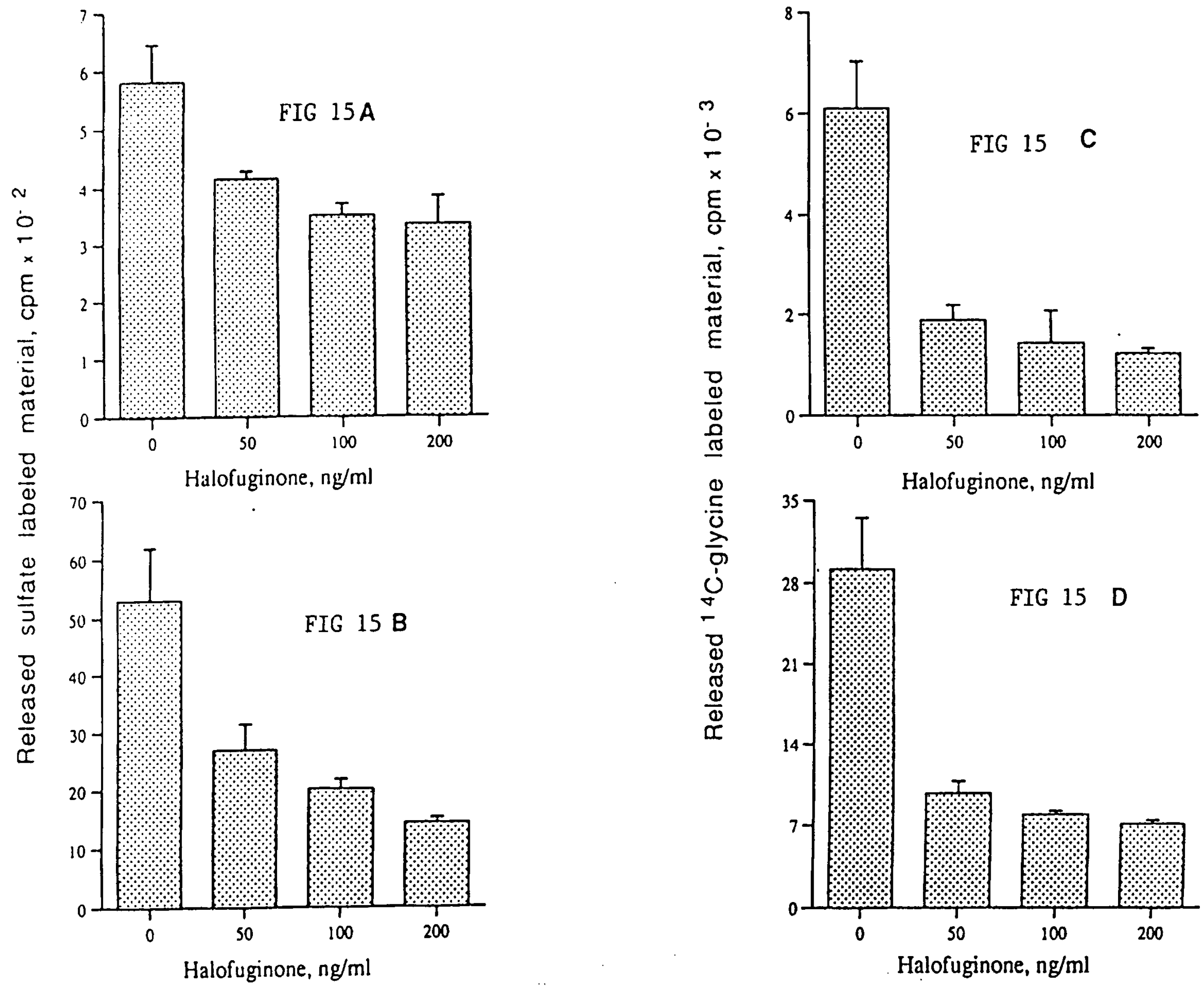

FIG16A

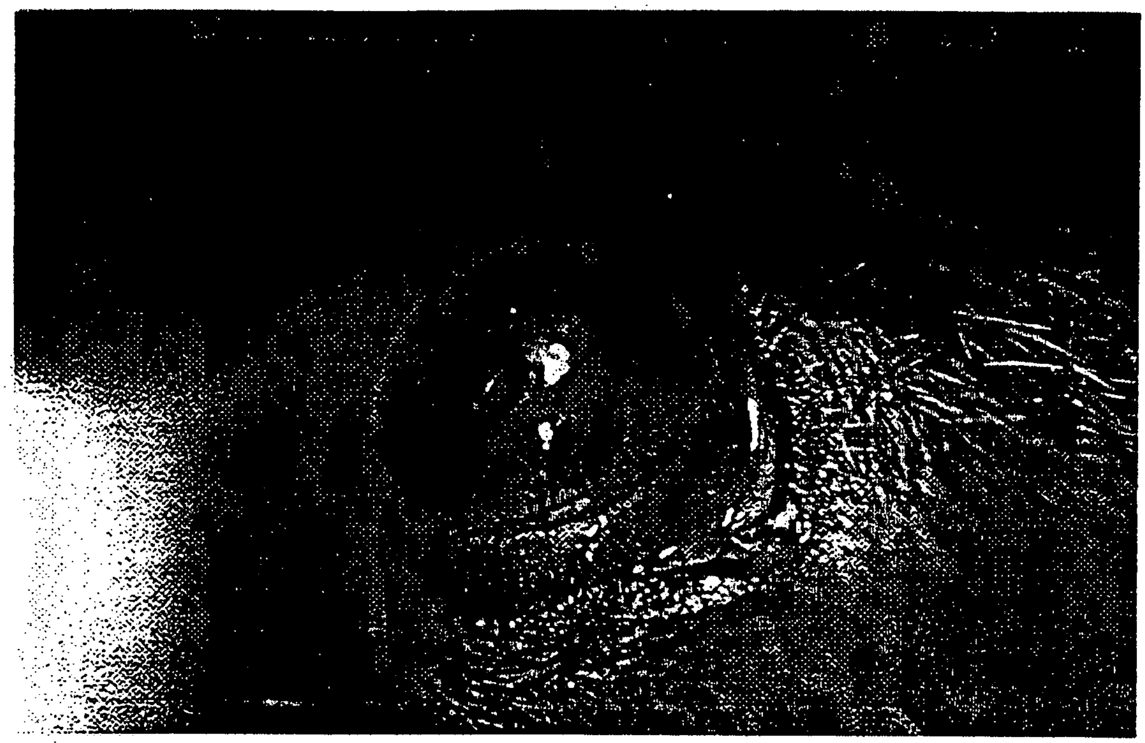

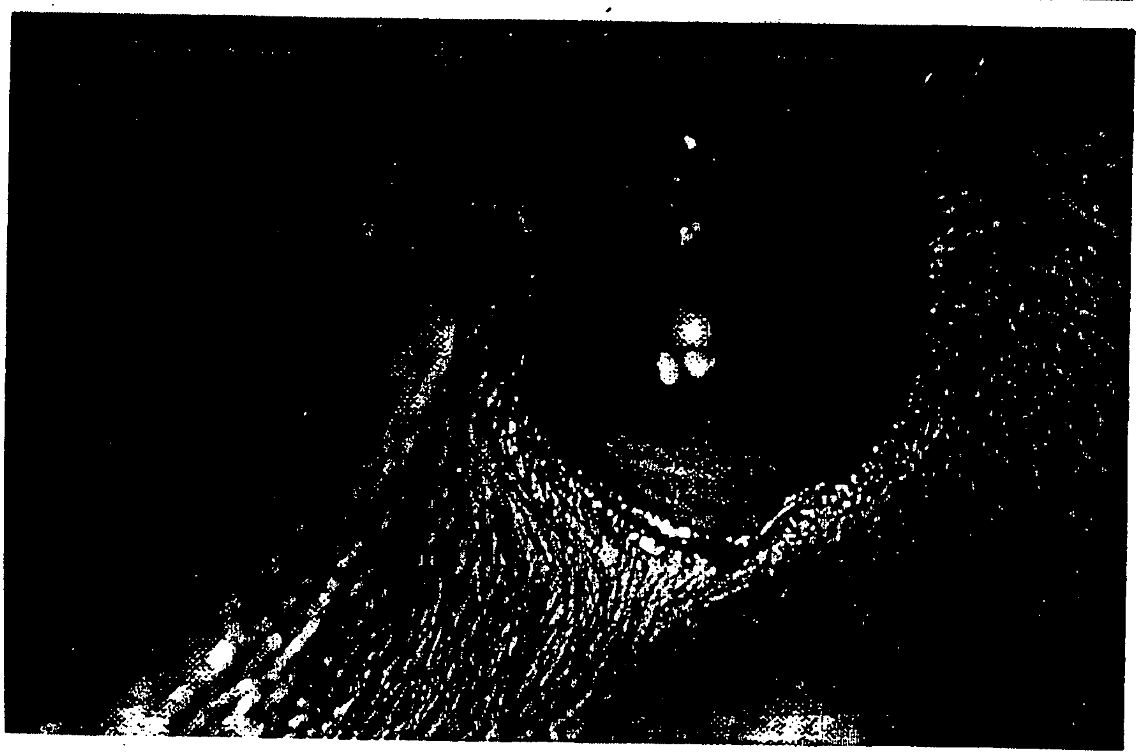

FIG 16 B

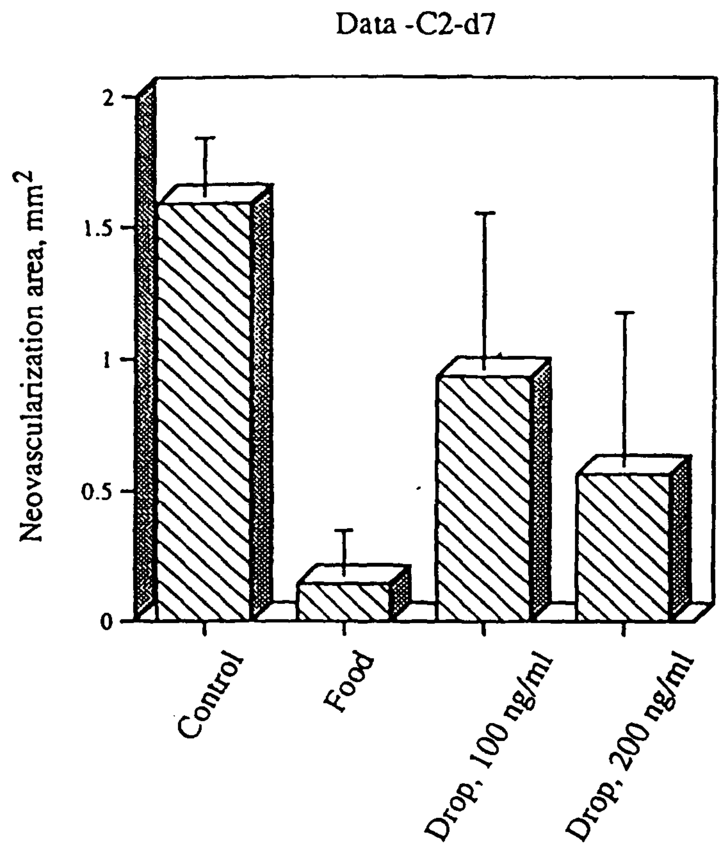
Data -C2-d7
Neovascularization area, mm$^2$
2
1.5
1
0.5
0
Control
Food
Drop, 100 ng/ml
Drop, 200 ng/ml

FIG 16 C

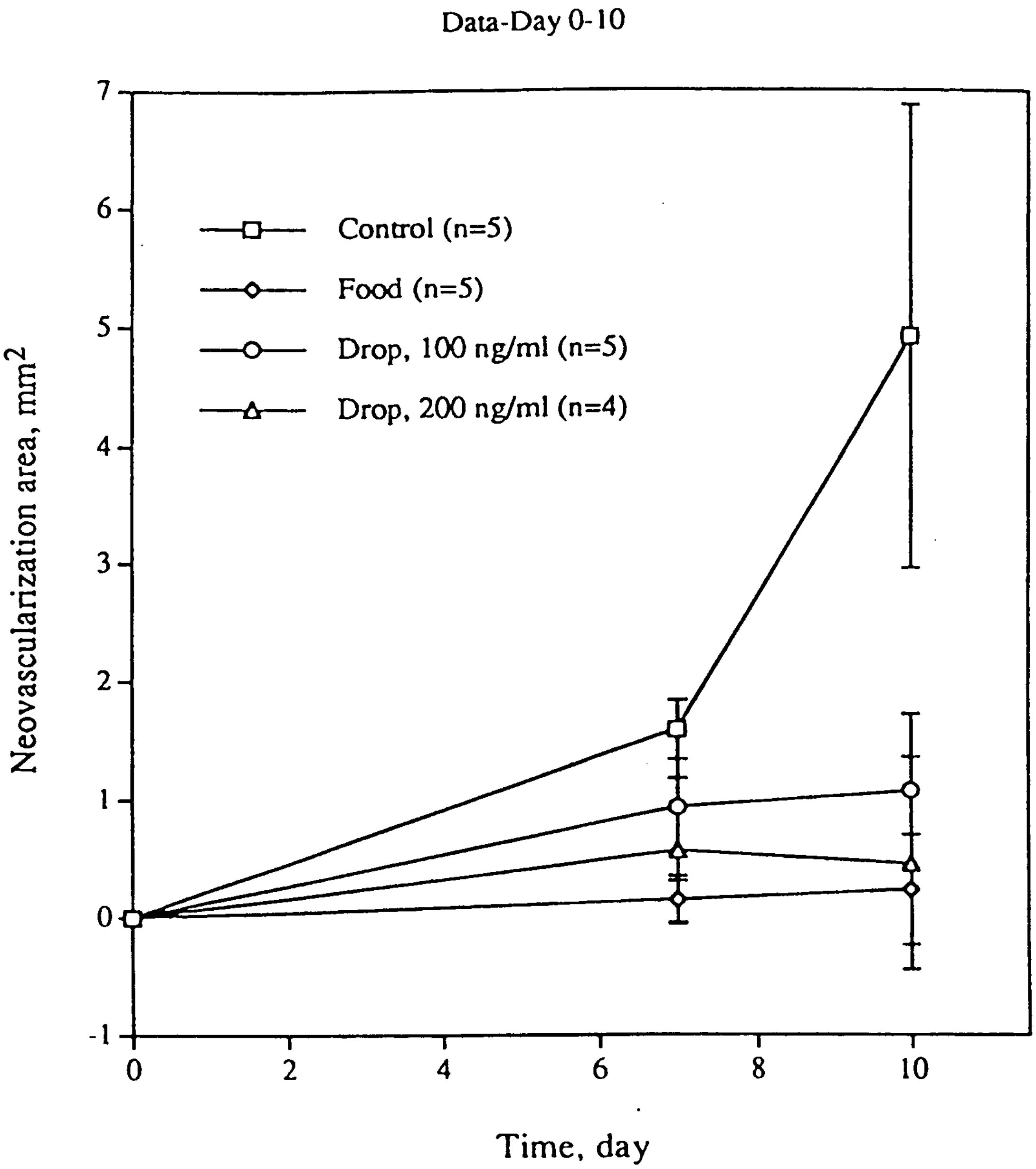
Data-Day 0-10
7
6
5
4
3
2
1
0
-1
0
2
4
6
8
10
Control (n=5)
Food (n=5)
Drop, 100 ng/ml (n=5)
Drop, 200 ng/ml (n=4)
Neovascularization area, mm2
Time, day
FIG 16 D

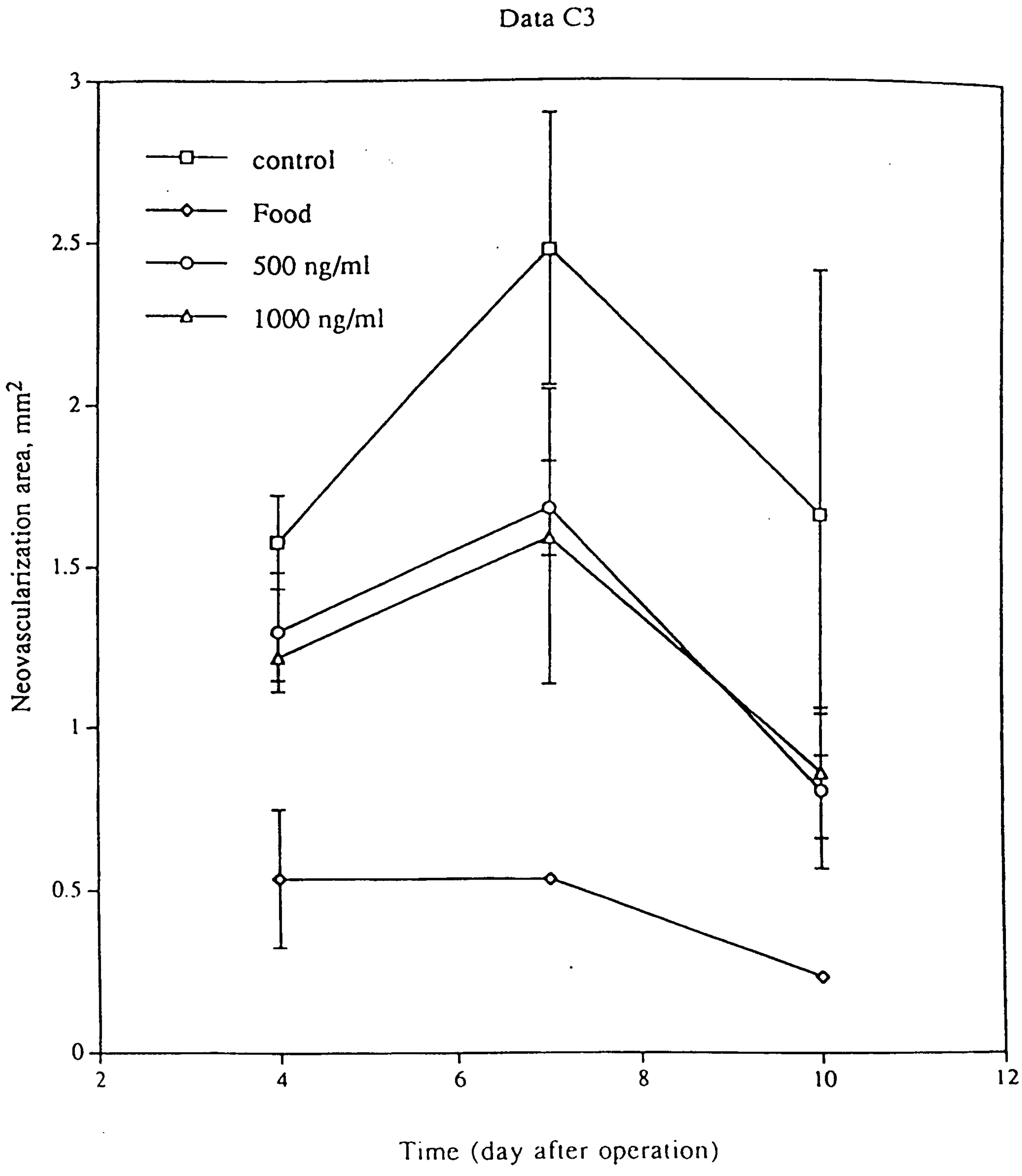
Data C3
control
Food
500 ng/ml
1000 ng/ml
Neovascularization area, mm2
0
0.5
1
1.5
2
2.5
3
2
4
6
8
10
12
Time (day after operation)
FIG 16 E

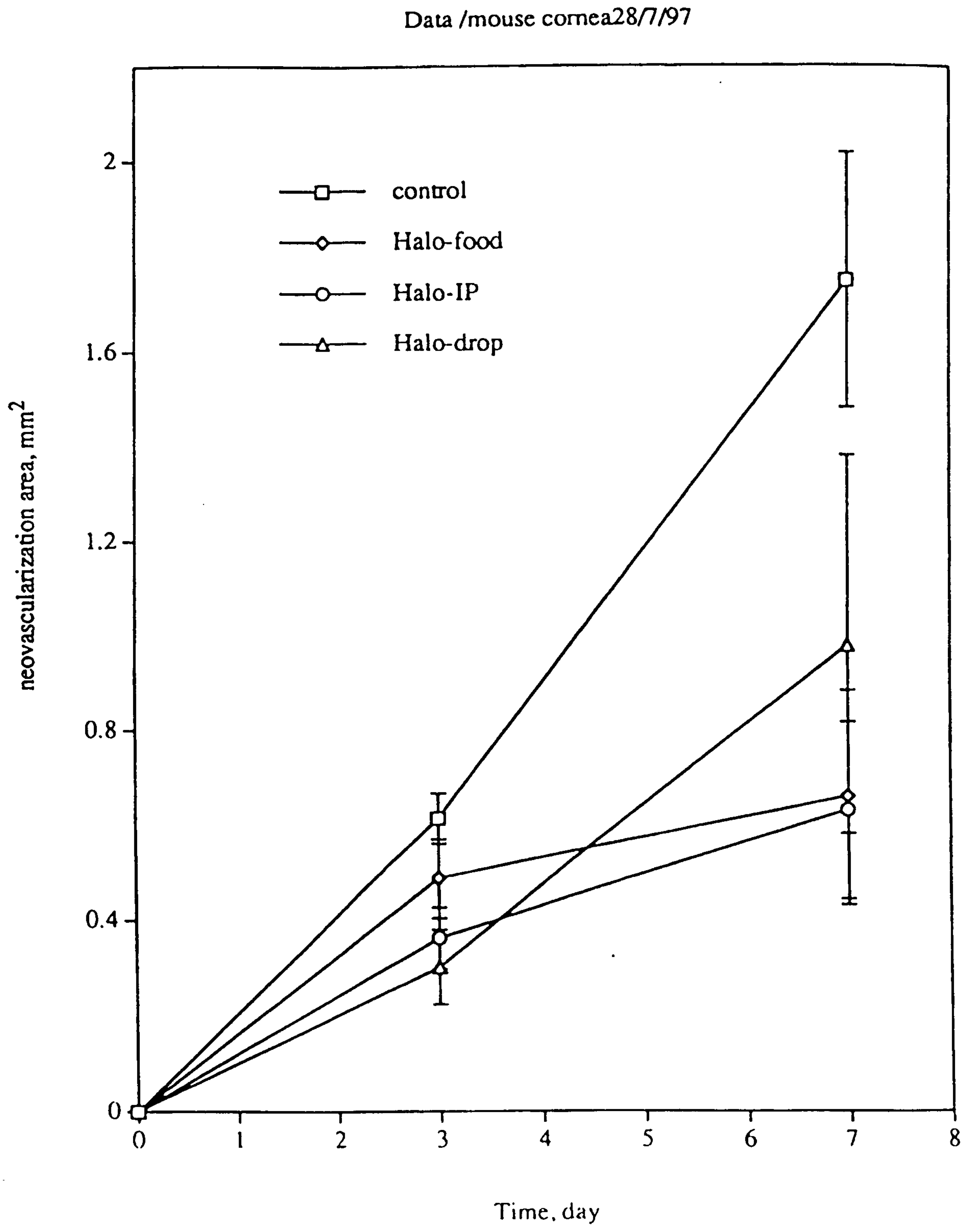
Data /mouse cornea28/7/97
control
Halo-food
Halo-IP
Halo-drop
neovascularization area, mm2
Time, day
0
0.4
0.8
1.2
1.6
2
0
1
2
3
4
5
6
7
8

FIG 16 F

# Effect of halofuginone on integrins

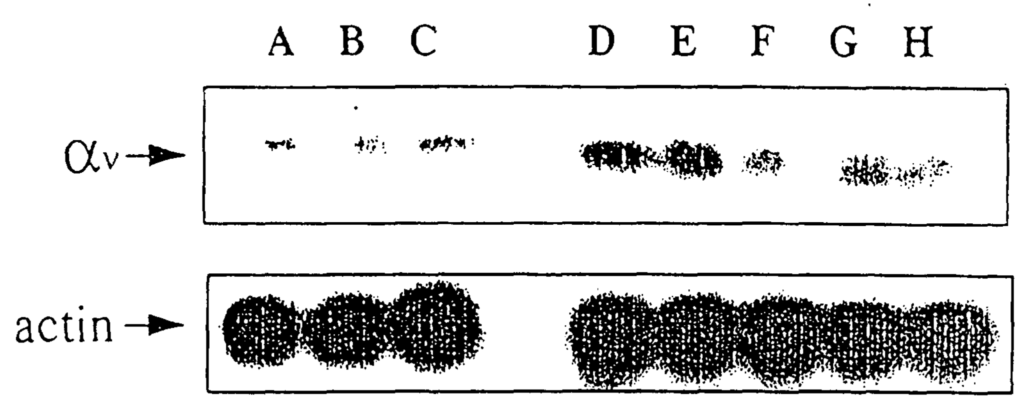


FIG 17

Effect of halofuginone on integrin gene expression

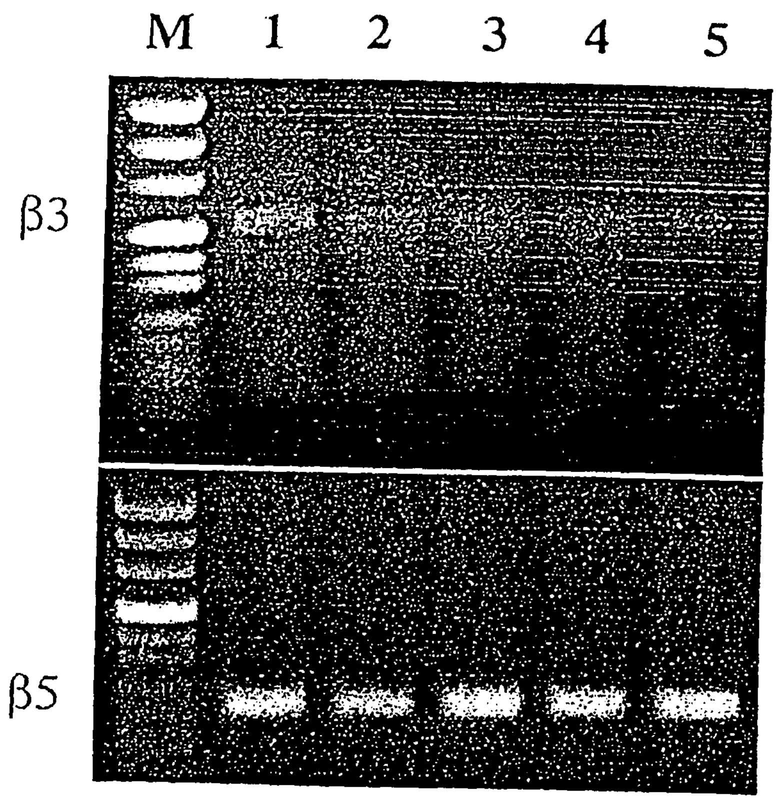


FIG 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5449678 A
- WO 9606616 A
- WO 9706805 A
- US 3320124 A
- US 3338909 A


### Non-patent literature cited in the description


- **J. FOLKMAN ; M. KLAGSBRUN.** *Science,* 1987, vol. 235, 442-447
- **J. FOLKMAN ; Y. SHING.** *J. Biol. Chem.,* 1992, vol. 267, 10931-10934
- **J. FOLKMAN.** *Perspect. in Biol. and Med,* 1985, vol. 29, 10-36
- **J. FOLKMAN.** *J. Natl. Cancer Inst,* 1989, vol. 82, 4-6
- **N. WEIDNER et al.** *Amer. J. Pathol,* 1993, vol. 143, 401-409
- **M.S. O'REILLY et al.** *Cell,* 1994, vol. 79, 316-328
- **J. FOLKMAN.** *ibid,* 1989
- **N. WEIDNER et al.** *ibid,* 1993
- **M.S. O'REILLY et al.** *ibid,* 1994
- **N. WEIDNER et al.** *N. Eng. J. Med,* 1991, vol. 324, 1-8
- **J. FOLKMAN.** *ibid,* 1987
- **J. FOLKMAN ; Y. SHING.** *ibid,* 1992
- **J. FOLKMAN.** *Nature Medicine,* 1995, vol. 1, 27-31
- **J.W. MILLER et al.** *J. Pathol,* 1994, vol. 145, 574-584
- **A.P. ADAMID et al.** *Amer. J. Ophthal,* 1994, vol. 118, 445-450
- **K. TAKAHASHI et al.** *J. Clin. Invest,* 1994, vol. 93, 2357-2364
- **D.J. PEACOCK et al.** *J. Exp. Med.,* 1992, vol. 175, 1135-1138
- **B.J. NICKOLOFF et al.** *Amer. J. Pathol,* 1994, vol. 44, 820-828
- **J. FOLKMAN.** Steroid Hormones and Uterine Bleeding. American Association for the Advancement of Science Press, 1992, 144-158
- **J. FOLKMAN.** *Perspectives in Biology and Medicine,* 1985, vol. 29, 1-36
- **J. BISCHOFF.** *Trends Cell Biol,* 1995, vol. 5, 69-74
- **M.L. IRUELA-ARISPE et al.** *Lab. Invest,* 1991, vol. 64, 174-186
- **C.J. JACKSON ; K.L. JENKINS.** *Exp. Cell Res.,* 1991, vol. 192, 319-323
- **MARAGOUDAKIS, M.E et al.** *Int. J. Radiat. Biol,* 1991, vol. 60, 54-9
- **NICOSIA, R.F et al.** *In Vitro Cell Dev. Biol.,* 1991, vol. 27
- **IRUELA-ARISPE, M.L et al.** *Arterioscler. Thromb,* 1991, vol. 11, 805-15
- **KITTELBERGER, R et al.** *Connect. Tissue Res,* 1990, vol. 24, 303-18
- **BROOKS, P.C et al.** *Cell,* 1994, vol. 79, 1157-1164
- **J. FOLKMAN.** *ibid,* 1995
- *J. Clin. Invest,* vol. 87, 1883-1888
- **D. INGBER et al.** *Nature,* vol. 348, 555-557
- **NAKAJIMA M et al.** *Cancer Res.,* 1989, vol. 9, 1698-1706
- **TURPEENNIEMI-HUJANEN, T et al.** *J. Natl. Cancer Inst,* 1985, vol. 75, 99-103
- **MONTEAGUDO, C et al.** *Amer. J. Pathol,* 1990, vol. 136, 585-592
- **HERRON, G.S et al.** *J. Biol. Chem.,* 1986, vol. 261, 2814-2818
- **REICH, R et al.** *Clin. and Exp. Metastasis,* 1995, vol. 13, 134-140
- **FOLKMAN, J.** *Seminars in Cancer Biology,* 1992, vol. 3, 56-71
- **DVORAK, H.F.** *N. Eng. J. Med,* 1986, vol. 315, 1650-1659
- Tumor Stroma. **YEO, T.K et al.** Diagnostic Immunopathology. Raven Press, 985-696
- **J.A. CASAS et al.** *Ann. Rhem. Dis,* 1987, vol. 46, 763
- **D. KERSHENOBICH et al.** *N. Engl. J. Med,* 1988, vol. 318, 1709
- *J. Biol Chem.,* 1990, vol. 265, 8414
- **C.J. CUNLIFFE et al.** *J. Med. Chem.,* 1992, vol. 35, 2652
- **T. SALO et al.** *J. Oral Pathol. Med,* 1990, vol. 19, 404
- **CHOI et al.** *Arch. Surg,* 1995, vol. 130, 257-261
- **B. TYCKO.** *Am. J. Path,* 1994, vol. 144, 431-439

- **DE GROOT, N et al.** *Trophoblast Res,* 1994, vol. 8, 2285-2302
- **RACHMILEWITZ, J et al.** *Oncogene,* 1995, vol. 11, 863-870
- **A. FERENCZY et al.** *Cancer,* 1971, vol. 28, 1004-1018
- **A. ZHANG et al.** *Endocrinology,* 1994, vol. 134, 1089-1094
- **R.S. MANGRULKER et al.** *Biology of Reproduction,* 1995, vol. 53, 636-646
- **I. VLODAVSKY et al.** Basement Membranes: Cellular and Molecular Aspects. Academic Press, Inc, 1993, 327-343
- **NAGLER, A et al.** *Arterioscler. Thromb. Vasc. Biol,* 1997, vol. 17, 194-202
- **HOLMGREN, L et al.** *Nat. Med,* 1995, vol. 1, 149-153
- **D, GOSPODAROWICZ et al.** *Proc. Natl. Acad. Sci. U.S.A.,* 1979, vol. 73, 4120
- **M. BENEZRA et al.** *Cancer Res.,* 1992, vol. 52, 5656-5662
- **I. VLODAVSKY et al.** *Proc. Natl. Acad. Sci. U.S.A.,* 1987, vol. 84, 2292-2296
- **R.F. NICOSA ; A. OTTINETTI.** *Lab. Invest,* 1990, vol. 63, 115-122
- **R. MONESANO et al.** *J. Cell Biol.,* 1983, vol. 97, 1648-1652
- **R.F. NICOSIA ; A. OTTINETTI.** *Lab. Invest,* 1990, vol. 63, 115-122
- **D. GOSPODAROWICZ et al.** *Exp. Eye Res,* 1977, vol. 25, 75-89
- **I. VLODAVSKY et al.** *Cancer Res.,* 1983, vol. 43, 2704-2711
- **I. VLODAVSKY et al.** *Proc. Natl. Acad. Sci. USA,* 1987, vol. 84, 2292-2296
- **KENYON, B.M et al.** *Invest. Ophthal. Visual,* 1996, vol. 37, 1625-1632
- **KENYON, B.M et al.** *Invest. Ophthal. Visual Sci,* 1996, vol. 37, 1625-1632
- **DRAKE, C.J et al.** *Dev. Dyn,* 1992, vol. 193, 83-91
- **BROOKS, P.C et al.** *Science,* 1994, vol. 264, 569-571
- **FELDING-HABERMANN, B et al.** *. Clin. Invest.,* 1992, vol. 89, 2018-2022